# EUROPEAN PATENT APPLICATION

(11) **EP 4 339 290 A1**
(43) Date of publication of application: **20.03.2024**
(21) Application number: 22806510.8
(22) Date of filing: 27.04.2022
(51) Int. Cl.: C12N 15/82, C12N 9/02, A01H 5/00

(54) **USE OF PROTOPORPHYRINOGEN OXIDASE**

(30) Priority: 12.05.2021 CN 202110514749
(71) Applicant: Beijing Dabeinong Biotechnology Co., Ltd., Beijing 100194 (CN)
(72) Inventor: XIAO, Xiang, Beijing 100193 (CN); SONG, Qingfang, Beijing 100193 (CN); TAO, Qing, Beijing 100193 (CN); YU, Caihong, Beijing 100193 (CN); BAO, Xiaoming, Beijing 100193 (CN)
(74) Representative: Nederlandsch Octrooibureau
(86) International application number: PCT/CN2022/089519
(87) International publication number: WO 2022/237541

(57) **Abstract**

The present invention relates to the use of a protoporphyrinogen oxidase. A method for controlling weeds comprises applying a herbicide containing an effective dose of a PPO inhibitor to a field in which at least one transgenic plant is present, wherein the transgenic plant comprises, in the genome thereof, a polynucleotide sequence encoding protoporphyrinogen oxidase, and the transgenic plant has reduced plant damage and/or has an increased plant yield compared with other plants that do not have a polynucleotide sequence encoding protoporphyrinogen oxidase. The protoporphyrinogen oxidase PPO 1-PPO 14 of the present invention has a high tolerance to a PPO inhibitor herbicide. In addition, the plants containing the polynucleotide sequence encoding protoporphyrinogen oxidase have a strong tolerance to the PPO-inhibitor herbicide, and show high-resistance tolerance to almost all of 4-fold field concentration of oxyfluorfen, saflufenacil and flumioxazin and 2-fold field concentration of sulfentrazone. Therefore, the protoporphyrinogen oxidase has broad application prospects in plants.

## Description

### FIELD OF THE INVENTION

The present invention relates to use of a protoporphyrinogen oxidase, and in particular to a method of confering tolerance to a PPO-inhibitor herbicide upon a plant using a protoporphyrinogen oxidase derived from prokaryotes, and use thereof.

### BACKGROUND

The biosynthetic pathway of porphyrin is used for synthesis of chlorophyll and heme, which play important roles in plant metabolism, and this pathway occurs in the chloroplast. In this pathway, protoporphyrinogen oxidases (PPOs) catalyze the oxidation of protoporphyrinogen IX to protoporphyrin IX. After protoporphyrin IX is produced, protoporphyrin IX is coupled to magnesium by magnesium chelatase to synthesize chlorophyll, or is coupled to iron by ferrochelatase to synthesize heme.

Herbicides that act by inhibiting PPOs mainly include PPO-inhibitor herbicides from the class of diphenyl ethers, oxadiazolones, N-phenylphthalimides, oxazolinones, phenylpyrazoles, uracils, thiadiazoles, triazolinones, triazinones, and others. In plants, PPO inhibitors inhibit the enzymatic activity of PPO, resulting in inhibition of synthesis of chlorophyll and heme, and an accumulation of the substrate protoporphyrinogen IX; the accumulated protoporphyrinogen IX is rapidly exported from the chloroplast to the cytoplasm, where protoporphyrinogen IX is converted to protoporphyrin IX in a non-enzymatic reaction, which, in the presence of light and oxygen molecules, further forms highly reactive singlet oxygen (¹O₂), which damages the cell membrane and rapidly leads to death of plant cells.

Methods for providing plants that are tolerant to PPO-inhibitor herbicides mainly include the following: 1) detoxifying the herbicide with an enzyme which transforms the herbicide or its active metabolite into non-toxic products; 2) overexpressing the sensitive PPO so as to produce quantities of the target enzyme in the plant which are sufficient in relation to the herbicide, in view of the kinetic constants of this enzyme, so as to have enough of the functional enzyme available despite the presence of its inhibitor; and 3) providing a functional PPO that is less sensitive to herbicides or their active metabolites but retains the capability of catalyzing the oxidation of protoporphyrinogen IX to protoporphyrin IX. With respect to functional PPOs, while a given functional PPO enzyme may provide a useful level of tolerance to some PPO-inhibitor herbicides, the same functional PPO may be quite inadequate to provide commercial levels of tolerance to a different, more desirable PPO-inhibitor herbicide. For example, PPO-inhibitor herbicides may differ in terms of the spectrum of weeds they control, their manufacturing costs, and their environmental benefits. Accordingly, new methods for conferring PPO-inhibitor herbicide tolerance upon various crops and crop varieties are needed.

### SUMMARY OF THE INVENTION

The object of the present invention is to provide use of a protoporphyrinogen oxidase. The protoporphyrinogen oxidase is derived from prokaryotes, and the plants transformed with a polynucleotide sequence encoding the protoporphyrinogen oxidase according to the present invention have good tolerance to PPO-inhibitor herbicides.

To achieve the above object, the present invention provides a method for controlling weeds, comprising applying a herbicide containing an effective dose of a PPO inhibitor to a field where at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats (*Avena sativa*)*,* wheat (*Triticum aestivum*)*,* barley (*Hordeum vulgare*)*,* millet (*Setaria italica*)*,* corn (*Zea mays*)*,* sorghum (*Sorghum bicolor*)*, Brachypodium distachyon,* rice (*Oryza sativa*)*,* tobacco (*Nicotiana tabacum*)*,* sunflower (*Helianthus annuus*)*,* alfalfa (*Medicago sativa*)*,* soybean (*Glycine max*)*, cicer arietinum,* peanut (*Arachis hypogaea*), sugar beet (*Beta vulgaris*)*,* cucumber (*Cucumis sativus*)*,* cotton (*Gossypium hirsutum*)*,* oilseed rape (*Brassica napus*)*,* potato (*Solanum tuberosum*)*,* tomato (*Solanum lycopersicum*) or *Arabidopsis thaliana,* further preferably, the transgenic plant is a glyphosate-tolerant plant, and the weeds are glyphosate-resistant weeds;
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

Preferably, the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.

Further, the transgenic plant further comprises at least one second polynucleotide encoding a second herbicide-tolerant protein, which is different from the polynucleotide sequence encoding the protoporphyrinogen oxidase.

The second polynucleotide encodes a selectable marker protein, a protein with synthetic activity, a protein with degradation activity, a biotic stress-resistant protein, an abiotic stress-resistant protein, a male sterility protein, a protein that affects plant yield and/or a protein that affects plant quality.

In particular, the second polynucleotide encodes 5-enolpyruvylshikimate-3-phosphate synthase, glyphosate oxidoreductase, glyphosate-N-acetyltransferase, glyphosate decarboxylase, glufosinate acetyltransferase, alpha-ketoglutarate-dependent dioxygenase, dicamba monooxygenase, 4-hydroxy phenyl pyruvate dioxygenase, acetolactate synthase, and/or cytochrome-like protein.

Alternatively, the herbicide containing an effective dose of a PPO inhibitor may further include glyphosate herbicides, glufosinate herbicides, auxin-like herbicides, graminicides, pre-emergence selective herbicides and/or post-emergence selective herbicides.

To achieve the above object, the present invention further provides a planting combination for controlling the growth of weeds, comprising a PPO-inhibitor herbicide and at least one transgenic plant, wherein the herbicide containing an effective dose of a PPO inhibitor is applied to a field where the at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase; wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato *or Arabidopsis thaliana;* further preferably, the transgenic plant is a glyphosate-tolerant plant, and the weeds are glyphosate-resistant weeds.
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

Preferably, the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.

Further, the transgenic plant further comprises at least one second polynucleotide encoding a second herbicide-tolerant protein, which is different from the polynucleotide sequence encoding the protoporphyrinogen oxidase.

The second polynucleotide encodes a selectable marker protein, a protein with synthetic activity, a protein with degradation activity, a biotic stress-resistant protein, an abiotic stress-resistant protein, a male sterility protein, a protein that affects plant yield and/or a protein that affects plant quality.

In particular, the second polynucleotide encodes 5-enolpyruvylshikimate-3-phosphate synthase, glyphosate oxidoreductase, glyphosate-N-acetyltransferase, glyphosate decarboxylase, glufosinate acetyltransferase, alpha-ketoglutarate-dependent dioxygenase, dicamba monooxygenase, 4-hydroxy phenyl pyruvate dioxygenase, acetolactate synthase, and/or cytochrome-like protein.

Alternatively, the herbicides containing an effective dose of a PPO inhibitor may further include glyphosate herbicides, glufosinate herbicides, auxin-like herbicides, graminicides, pre-emergence selective herbicides and/or post-emergence selective herbicides.

To achieve the above object, the present invention further provides a method for generating a plant which is tolerant to a PPO-inhibitor herbicide, wherein the method comprises introducing a polynucleotide sequence encoding a protoporphyrinogen oxidase into the genome of the plant, and when the herbicide comprising an effective dose of a PPO inhibitor is applied to a field where at least the plant is present, the plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the introduction method comprises genetic transformation, genome editing or gene mutation methods;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato *or Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

To achieve the above object, the present invention further provides a method for cultivating a plant which is tolerant to a PPO-inhibitor herbicide, comprising:
planting at least one plant propagule, wherein the plant propagule comprises in its genome a polynucleotide encoding a protoporphyrinogen oxidase, wherein the protoporphyrinogen oxidase has at least 88% sequence identity to an amino acid sequence selected from the group consisting of SEQ ID NOs: 1-14;
allowing the plant propagule to grow into a plant; and
applying the herbicide comprising an effective dose of a PPO inhibitor to a field comprising at least the plant, and harvesting the plant having a reduced plant damage and/or increased plant yield compared with other plants without the polynucleotide sequence encoding the protoporphyrinogen oxidase;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato *or Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

To achieve the above object, the present invention further provides a method for protecting a plant from damages caused by a PPO-inhibitor herbicide or for conferring tolerance to the PPO-inhibitor herbicide upon a plant, comprising applying the herbicide comprising an effective dose of a PPO inhibitor to a field where at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

To achieve the above object, the present invention further provides use of a protoporphyrinogen oxidase for conferring tolerance to a PPO-inhibitor herbicide upon a plant, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the use of the protoporphyrinogen oxidase for conferring tolerance to a PPO-inhibitor herbicide upon a plant comprises applying the herbicide containing an effective dose of a PPO inhibitor to a field where at least one transgenic plant is present, wherein the transgenic plant comprises in its genome a polynucleotide sequence encoding the protoporphyrinogen oxidase, and the transgenic plant has a reduced plant damage and/or increased plant yield compared with other plants without the polynucleotide sequence encoding the protoporphyrinogen oxidase;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato *or Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

Preferably, the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.

As a specific embodiment, the PPO-inhibitor herbicide (also known as herbicide of the PPO inhibitor class) may be one or more selected from the group consisting of, but is not limited to: diphenyl ethers (chlornitrofen, chlomethoxyfen, bifenox, oxyfluorfen, acifluorfen, its salts and esters, fomesafen, lactofen, fluoroglycofen-ethyl, ethoxyfen-ethyl, aclonifen, bifenox, ethoxyfen, chlorintrofen and halosafen); oxadiazolones (oxadiazon, and oxadiargyl); N-phenylphthalimides (flumioxazin, flumiclorac-pentyl and cinidon-ethyl); oxazolinones (pentoxazone); phenylpyrazoles (fluazolate and pyraflufen-ethyl); uracils (benzfendizone, butafenacil and saflufenacil); thiadiazoles (thidiazimin and fluthiacet); triazolinones (azafenidin, sulfentrazone and carfentrazone); triazinones (trifludimoxazin); and others (flufenpyr-ethyl and pyraclonil).

As used herein, the articles "a" and "an" refer to one or more than one (i.e., to at least one). For example, "an element" means one or more elements (components). Furthermore, the term "comprise" or variants thereof such as "comprises" or "comprising" should be understood to imply the inclusion of a stated element, integer or step, or group of elements, integers or steps, but not the exclusion of any other element, integer or step, or group of elements, integers or steps.

As used herein, the term "herbicide-insensitive" means the ability of a protoporphyrinogen oxidase to maintain at least some of its enzymatic activity in the presence of one or more PPO herbicide(s). Enzymatic activity of a protoporphyrinogen oxidase can be measured by any means known in the art, for example, by an assay in which the production of the product of protoporphyrinogen oxidase or the consumption of the substrate of protoporphyrinogen oxidase in the presence of one or more PPO herbicide(s) is measured via fluorescence, high performance liquid chromatography (HPLC), or mass spectrometry (MS). "Herbicide-insensitivity" may be complete or partial insensitivity to a particular herbicide, and may be expressed as a percent (%) tolerance or insensitivity to a particular PPO herbicide.

As used herein, the term "herbicide tolerance of plants, seeds, plant tissues or cells" or "herbicide-tolerant plants, seeds, plant tissues or cells" refers to the ability of plants, seeds, plant tissues or cells to resist the effect of applied herbicides. For example, herbicide-tolerant plants can survive or continue to grow in the presence of the herbicide. Herbicide tolerance of a plant, seed, plant tissue or cell can be measured by comparing the plant, seed, plant tissue or cell with an appropriate control. For example, herbicide tolerance can be measured and assessed by applying the herbicide to a plant (test plant) which comprises a DNA molecule encoding a protein capable of conferring herbicide tolerance, and to a plant (control plant) which does not comprise a DNA molecule encoding a protein capable of conferring herbicide tolerance, and then comparing the damage of the two plants, wherein the herbicide tolerance of the test plant is indicated by a reduction in the damage rate compared with the control plant. The herbicide-tolerant plant, seed, plant tissue or cell exhibits a reduced response to the toxic effects of the herbicide as compared with the control plant, seed, plant tissue or cell. The term "herbicide tolerance trait" is a transgenic trait imparting improved herbicide tolerance to a plant as compared with the wild-type plant. Plants which might be produced with an herbicide tolerance trait of the present invention could include, for instance, any plant including crop plants such as oats, wheat, barley, millet, corn, sorghum, *Brachypodium distachyon,* rice, tobacco, sunflower, alfalfa, soybean, *Cicer arietinum,* peanuts, sugar beet, cucumber, cotton, oilseed rape, potato, tomato and *Arabidopsis thaliana.*

DNA molecules of the present invention may be synthesized and modified by methods known in the art, either completely or in part, especially where it is desirable to provide sequences useful for DNA manipulation (such as restriction enzyme recognition sites or recombination-based cloning sites), plant-preferred sequences (such as plant-codon usage or Kozak consensus sequences), or sequences useful for DNA construct design (such as spacer or linker sequences). The present invention includes DNA molecules, preferably proteins, encoding the protein having at least 88% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, and at least 99% sequence identity to an amino acid sequence selected from the group consisting of SEQ ID NOs: 1-14. The term "percent sequence identity" or "% sequence identity" refers to the percentage of identical amino acids in a protein sequence of a reference sequence or query sequence (or its complementary strand) as compared to a test sequence (or its complementary strand) when the two sequences are aligned. Methods of alignment of sequences are well-known in the art and can be accomplished using mathematical algorithms such as the algorithm of Myers and Miller (1988) CABIOS 4:11-17; the local alignment algorithm of Smith et al. (1981) Adv. Appl. Math. 2:482; the global alignment algorithm of Needleman and Wunsch (1970) J. Mol. Biol. 48:443-453; and the algorithm of Karlin and Altschul (1990) Proc. Natl. Acad. Sci. USA 872264, modified as in Karlin and Altschul (1993) Proc. Natl. Acad. Sci. USA 90:5873-5877. Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTAin the GCG Wisconsin Genetics Software Package, Version 10 (available from Accelrys Inc., 9685 Scranton Road, San Diego, California, USA). Percent sequence identity is represented as the identity fraction multiplied by 100.

As used herein, oxyfluorfen refers to 2-chloro-1-(3-ethoxy-4-nitrophenoxy)-4-trifluoromethylbenzene as a colorless crystal solid. It is an ultra-low dosage selective, pre- and post-emergence contact PPO-inhibitor herbicide of diphenyl ethers, and can be applied as an emulsifiable concentrate. Weeds are killed mainly by absorbing the herbicide through coleoptile and mesocotyl. Oxyfluorfen can effectively control weeds in fields of rice, soybean, corn, cotton, vegetables, grapes, fruit trees and other crops. The weeds which can be controlled comprise, but are not limited to, monocotyledon and broad-leaf weeds of *Barnyard grass, Sesbania cannabina, Bromus tectorum, Setaria viridis, Datura stramonium, Agropyron repens, Ambrosia artemisiifolia, Sida spinosa, Abutilon theophrasti* and *Brassica kaber.*

As used herein, the effective dose of oxyfluorfen means being used at an amount ranging from 180 to 720 g ai/ha, including from 190 to 700 g ai/ha, from 250 to 650 g ai/ha, from 300 to 600 g ai/ha or from 400 to 500 g ai/ha.

As used herein, saflufenacil refers to N'- [2-chloro-4-fluoro-5-(3-methyl-2,6-dioxo-4-(trifluoromethyl)-3,6-dihydro-1(2H)-pyrimidine) benzoyl]-N-isopropyl-N-methylsulfamide as a light brown extruded granular solid. It belongs to sterilant PPO-inhibitor herbicides of uracils, and can be made into 70% water-dispersible granules. Saflufenacil is effective against a variety of broad-leaf weeds, including those resistant to glyphosate, ALS, and triazines, and is characterized by rapid killing effect and rapid residue degradation in soil.

As used herein, the effective dose of saflufenacil means being used at an amount ranging from 25 to 100 g ai/ha, including from 30 to 95 g ai/ha, from 40 to 90 g ai/ha, from 50 to 85 g ai/ha or from 60 to 80 g ai/ha.

As used herein, flumioxazin refers to 2-[7-fluoro-3,4-dihydro-3-oxo-4-(2-propynyl)-2H-1,4-benzoxazin-6-yl]-4,5,6,7-tetrahydro-1H-isoindole-1,3(2H)-dione. It is a PPO-inhibitor herbicide of N-phenylphthalimides which is absorbed by seedlings and leaves and is typically applied in a dosage form of 50% wettable powder and 48% suspension concentrate. Flumioxazin can effectively control annual broad-leaf weeds and some gramineous weeds. It is easily degraded in the environment and is safe for succeeding crops.

As used herein, the effective dose of flumioxazin means being used at an amount ranging from 60 to 240 g ai/ha, including from 70 to 220 g ai/ha, from 85 to 200 g ai/ha, from 90 to 185 g ai/ha or from 100 to 150 g ai/ha.

As used herein, sulfentrazone refers to N-(2,4-dichloro-5-(4-difluoromethyl- 4,5-dihydro-3-methyl-5-oxo-1H-1,2,4-triazole-1-yl)phenyl)methanesulfonamide as a tan solid. It is a PPO-inhibitor herbicide of triazolinones in a tipical dosage form of 38.9% and 44.5% suspension concentrate. Sulfentrazone can be used for controlling, among others, annual broad-leaf weeds, gramineous weeds and Cyperaceae such as *Ipomoea nil, Amaranthus retroflexus, Chenopodium album, Datura stramonium, Digitaria sanguinalis, Setaria viridis, Xanthium strumarium, Eleusine indica Gaertn, Cyperus rotundus* and the like in fields of corn, sorghum, soybean, peanut and the like.

As used herein, the effective dose of sulfentrazone means being used at an amount ranging from 450 to 900 g ai/ha, including from 500 to 850 g ai/ha, from 550 to 700 g ai/ha, from 500 to 685 g ai/ha or from 500 to 650 g ai/ha.

As used herein, the term "resistance" is inheritable and allows a plant to grow and propagate under the circumstance where an effective treatment with an ordinary herbicide is performed on a given plant. As recognized by a person skilled in the art, even if there is certain degree of damage (such as small necrosis, dissolution, chlorosis or other damage) to the given plant treated with the herbicide, at least the yield is not significantly compromised and thus the plant can still be considered as "resistant". In other words, the given plant has increased ability to resist various degrees of damage induced by the herbicide, and in general, damage to a wild-type plant with the same genotype can be caused at the same dose of the herbicide. The term "tolerant" or "tolerance" in the present invention is more extensive than the term "resistance" and includes "resistance".

As used herein, the biotic stress-resistant proteins refer to proteins that resist stress imposed by other organisms, including insect resistant-proteins, and (viral, bacterial, fungal and nematode) disease-resistant proteins.

As used herein, the abiotic stress-resistant proteins refer to proteins that resist stress imposed by the external environment, including proteins which are tolerant to herbicides, dryness, heat, chill, frost, salt stress, oxidative stress and the like.

As used herein, the proteins that affect plant quality refer to proteins that affect plant output traits, such as proteins that improve the quality and content of starch, oil, vitamins and the like, and proteins that improve fiber quality.

In addition, the expression cassette comprising a polynucleotide sequence encoding a protoporphyrinogen oxidase may also be expressed together with at least one protein encoding a herbicide-tolerant gene in plants. The herbicide-tolerant protein includes, but is not limited to, 5-enolpyruvylshikimate-3-phosphate synthase (EPSPS), glyphosate oxidoreductase (GOX), glyphosate-N-acetyltransferase (GAT), glyphosate decarboxylase, glufosinate acetyltransferase (PAT), alpha-ketoglutarate-dependent dioxygenase (AAD), dicamba monooxygenase (DMO), 4-hydroxy phenyl pyruvate dioxygenase (HPPD), acetolactate synthase (ALS), and/or cytochrome-like protein (P450).

As used herein, "glyphosate" refers to N-phosphonomethylglycine and salts thereof, and the treatment with "a glyphosate herbicide" refers to the treatment with any herbicide formulation containing glyphosate. Commercial formulations of glyphosate include, but are not limited to, ROUNDUP^{®} (glyphosate as an isopropylammonium salt), ROUNDUP^{®}WEATHERMAX (glyphosate as a potassium salt); ROUNDUP^{®}DRY and RIVAL^{®} (glyphosate as an ammonium salt); ROUNDUP^{®}GEOFORCE (glyphosate as a sodium salt); and TOUCHDOWN^{®} (glyphosate as a trimethylsulfonium salt).

As used herein, the effective dose of glyphosate means being used at an amount ranging from 200 to 1,600 g ae/ha, including from 250 to 1,600 g ae/ha, from 300 to 1,600 g ae/ha, from 500 to 1,600 g ae/ha, from 800 to 1,500 g ae/ha, from 1000 to 1,500 g ae/ha, from 800 to 1,500 g ae/ha.

As used herein, "glufosinate", also known as phosphinothricin, refers to ammonium 2-amino-4-[hydroxy(methyl)phosphoryl]butanoic acid, and the treatment with "a glufosinate herbicide" refers to the treatment with any herbicide formulation containing glufosinate.

As used herein, the effective dose of glufosinate means being used at an amount ranging from 200 to 800 g ae/ha, including from 200 to 750 g ae/ha, from 250 to 700 g ae/ha, from 300 to 700 g ae/ha, from 350 to 650 g ae/ha or from 400 to 600 g ae/ha.

In the present invention, the auxin-like herbicides mimic or act like the natural plant growth regulators called auxins. They affect cell wall plasticity and nucleic acid metabolism, which can lead to uncontrolled cell division and growth. The injury symptoms caused by auxin-like herbicides include epinastic bending and twisting of stems and petioles, leaf cupping and curling, and abnormal leaf shape and venation. Auxin-like herbicides include, but are not limited to, a phenoxy carboxylic acid compound, benzoic acid compound, pyridine carboxylic acid compound, quinoline carboxylic acid compound, and benazoli nethyl compound. Typically, the auxin-like herbicide is dicamba, 2,4-dichlorophenoxyacetic acid (2,4-D), (4-chloro-2-methylphenoxy)acetic acid (MCPA) and/or 4-(2,4-dichlorophenoxy)butyric acid (2,4-DB).

As used herein, "dicamba" refers to 3,6-dichloro-o-anisic acid or 3,6-dichloro-2-methoxy benzoic acid and its acids and salts. Its salts include isopropylamine, diglycoamine, dimethylamine, potassium and sodium salts. Commercial formulations of dicamba include, but are not limited to, Banvel^{®} (as DMA salt), Clarity^{®} (as DGA salt, BASF), VEL-58-CS-11^{™} and Vanquish^{®} (as DGA salt, BASF).

The 2,4-D in the present invention is a broad-spectrum, relatively inexpensive and powerful broad-leaved herbicide, which has been used for broad-spectrum broad-leaved weed control for more than 65 years under agricultural and non-crop conditions. 2,4-D has different levels of selectivity to different plants (for example, dicotyledonous plants are more sensitive than gramineous plants). Plants usually metabolize 2,4-D slowly, so different activities of target sites are more likely to explain different responses of plants to 2,4-D. Plant metabolism of 2,4-D is generally realized by two steps of metabolism, i.e., usually hydroxylation followed by conjugation with amino acids or glucose.

The selective herbicides before germination in the present invention include, but are not limited to, acetanilide, acetochlor, acetolactate synthase inhibitor and dinitroaniline.

The selective herbicides after germination in the present invention include, but are not limited to, nicosulfuron, rimsulfuron, and quizalofop-p-ethyl.

The application amount of the herbicide in the present invention varies with the soil structure, pH value, organic matter content, farming system and size of the weeds, and is determined by checking the appropriate application amount of the herbicide on the herbicide label.

As used herein, the term "confer" refers to providing a characteristic or trait, such as herbicide tolerance and/or other desirable traits to a plant.

As used herein, the term "heterologous" means from another source. In the context of DNA, "heterologous" refers to any foreign "non-self" DNA including that from another plant of the same species. For example, in the present invention a soybean HPPD gene that can be expressed in a soybean plant by means of transgenic methods would still be considered as "heterologous" DNA.

As used herein, the term "nucleic acid" includes a deoxyribonucleotide or ribonucleotide polymer in either single- or double-stranded forms, and unless otherwise specified, encompasses known analogues (e.g., peptide nucleic acids) having the essential properties of natural nucleotides in that they hybridize to single-stranded nucleic acids in a manner similar to naturally occurring nucleotides.

As used herein, the term "encoding" or "encoded" when used in the context of a specified nucleic acid means that the nucleic acid comprises the requisite information to direct translation of the nucleotide sequence into a specified protein. The information by which a protein is encoded is specified by the use of codons. A nucleic acid encoding a protein may comprise non-translated sequences (e.g., introns) within translated regions of the nucleic acid or may lack such intervening non-translated sequences (e.g., as in cDNA).

DNA sequences encoding the protoporphyrinogen oxidase of the present invention are used in the provision of plants, plant cells and seeds of the present invention that offer better tolerance to multiple PPO-inhibitor herbicides compared with like plants (control plants) which donot contain the DNA sequence encoding the protoporphyrinogen oxidase of the present invention.

The genes encoding the protoporphyrinogen oxidase of the present invention are useful for generating plants tolerant to PPO-inhibitor herbicides. The genes encoding the protoporphyrinogen oxidase of the present invention are particularly suitable for expression in plants to confer herbicide tolerance upon plants.

The terms "polypeptide", "peptide", and "protein" are used interchangeably herein and refer to a polymer of amino acid residues. The terms apply to amino acid polymers in which one or more amino acid residues are an artificial chemical analogue of a corresponding naturally occurring amino acid, as well as to naturally occurring amino acid polymers. Polypeptides of the invention can be produced either from a nucleic acid disclosed herein, or by means of standard molecular biology techniques. For example, a truncated protein of the invention can be produced by expression of a recombinant nucleic acid of the invention in an appropriate host cell, or alternatively by a combination of *ex vivo* procedures, such as protease digestion and purification.

The present invention also provides nucleic acid molecules comprising the protoporphyrinogen oxidase. In general, the present invention includes any polynucleotide sequence encoding a protoporphyrinogen oxidase having one or more conservative amino acid substitutions relative to the protoporphyrinogen oxidase. Conservative substitutions providing functionally similar amino acids are well-known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (I), Lysine (K), Histidine (H); Acidic: Aspartic acid (D), Glutamic acid (E), Asparagine (N), Glutamine (Q).

Accordingly, sequences which have tolerance activity to a protoporphyrinogen oxidase inhibitor herbicide and hybridize to genes encoding protoporphyrinogen oxidase of the present invention under strict conditions are included in the present invention. Exemplary sequences comprise at least about 85%, 90%, 91%, 92%, 93%, 94%, 95%, 96%, 97%, 98%, 99% or more sequence identity to SEQ ID NO:29-42 and SEQ ID NO:62-64 of the invention. The genes encoding the protoporphyrinogen oxidase of the present invention do not include SEQ ID NO: 15-28.

Any conventional nucleic acid hybridization or amplification method can be used to identify the presence of the PPO gene of the present invention. A nucleic acid molecule or a fragment thereof is capable of specifically hybridizing to other nucleic acid molecules under certain circumstances. In the present invention, if two nucleic acid molecules can form an anti-parallel double-stranded nucleic acid structure, then it can be considered that these two nucleic acid molecules can be specifically hybridized to each other. If two nucleic acid molecules exhibit a complete complementarity, then one nucleic acid molecule of the two is said to be the "complement" of the other nucleic acid molecule. In the present invention, when each nucleotide of a nucleic acid molecule is complementary to the corresponding nucleotide of another nucleic acid molecule, then these two nucleic acid molecules are said to exhibit a "complete complementarity". If two nucleic acid molecules can be hybridized to each other with a sufficient stability to allow them to anneal and bind with each other at least under conventional "low stringency" conditions, then these two nucleic acid molecules are said to be "minimally complementary". Similarly, if two nucleic acid molecules can be hybridized to each other with a sufficient stability to allow them to anneal and bind with each other under conventional "high stringency" conditions, then these two nucleic acid molecules are said to be "complementary". Deviation from a complete complementarity is permissible, as long as this deviation does not completely prevent two molecules from forming a double-stranded structure. In order to enable a nucleic acid molecule to act as a primer or probe, it is only guaranteed that the molecule has a sufficient complementarity in its sequence to allow a stable double-stranded structure to be formed under the conditions of particular solvent and salt concentration.

In the present invention, a substantially homologous sequence is a nucleic acid molecule that can be specifically hybridized to the complementary strand of a matched nucleic acid molecule under high stringency conditions. Suitable stringent conditions that promote DNA hybridization are well-known to a person skilled in the art; for example, the suitable stringent conditions can be achieved by treating with 6.0× sodium chloride/sodium citrate (SSC) under conditions of approximately 45 °C, and then washing with 2.0×SSC under conditions of 50 °C. For example, the salt concentration in the washing step can be selected from the low stringency condition of about 2.0×SSC and 50 °C to the high stringency condition of about 0.2×SSC and 50 °C. In addition, the temperature condition in the washing step can rise from the low stringency condition of room temperature (about 22 °C) to the high stringency condition of about 65 °C. The temperature condition and the salt concentration can both vary, and it is also possible that one of the two remains unchanged, while the other varies. Preferably, the stringent conditions in the present invention can be achieved by specifically hybridizing to the genes encoding the protoporphyrinogen oxidase of the present invention in a 6×SSC, 0.5% SDS solution at 65 °C, and then washing the membrane each once with 2×SSC, 0.1% SDS and 1×SSC, 0.1% SDS.

As used herein, the term "hybridizing" or "hybridizing specifically" refers to the binding, duplexing, or hybridizing of a molecule only to a particular polynucleotide sequence under stringent conditions when that sequence is present in a complex mixture (e.g., total cellular) DNA or RNA.

Because of the degeneracy of the genetic codon, a variety of different DNA sequences may encode the same amino acid sequence. It is within the skill of a person skilled in the art to produce these alternative DNA sequences encoding the same or substantially the same protein. These different DNA sequences are included in the scope of the present invention. The "substantially the same" sequence refers to a sequence with an amino acid substitution, deletion, addition or insertion that does not substantively affect the herbicide tolerance activity, and includes a fragment retaining the herbicide tolerance activity.

The term "functional activity" or "activity" in the present invention means that the protein/enzyme used in the present invention (alone or in combination with other proteins) has the ability to degrade an herbicide or diminish the herbicide activity. A plant producing the protein of the present invention preferably produces an "effective amount" of the protein, so that when treating the plant with an herbicide, the protein expression level is sufficient to confer the plant a complete or partial tolerance to the herbicide (unless otherwise specified, in a general amount). The herbicide can be used in an amount which would usually kill a target plant or in a normal field amount and concentration. Preferably, the plant cell and plant of the present invention are protected from growth suppression or damage caused by treatment with the herbicide. The transformed plant and plant cell of the present invention are preferably tolerant to PPO-inhibitor herbicides, that is, the transformed plant and plant cell can grow in the presence of an effective dose of PPO-inhibitor herbicides.

The gene and protein in the present invention not only comprise a specific exemplary sequence, but also comprise a portion and/or a fragment (including an internal deletion and/or terminal deletion compared to the full-length protein), a variant, a mutant, a variant protein, a substitute (a protein having substituted amino acids), a chimera and a fusion protein which retain the activity characteristic of the specific exemplary protein.

The term "variant" in the present invention is intended to mean substantially similar sequences. For polynucleotides, a variant comprises a deletion and/or addition of one or more nucleotides at one or more internal sites within the reference polynucleotide and/or a substitution of one or more nucleotides at one or more sites in the herbicide tolerance gene. As used herein, the term "reference polynucleotide or polypeptide" comprises a herbicide tolerant polynucleotide sequence or amino acid sequence, respectively. As used herein, the term "native polynucleotide or polypeptide" comprises a naturally occurring polynucleotide sequence or amino acid sequence, respectively. For nucleic acid molecules, conservative variants include polynucleotide sequences that, because of the degeneracy of the genetic code, encode one of the protoporphyrinogen oxidases of the present invention. Naturally occurring allelic variants such as these can be identified with the use of well-known molecular biology techniques, for example, with polymerase chain reaction (PCR) and hybridization techniques as outlined below. Variant nucleic acid molecules also include synthetically derived nucleic acid molecules, such as those generated, for example, by using site-directed mutagenesis but which still encode a nucleotide sequence of the protoporphyrinogen oxidase of the invention. Generally, variants of a particular nucleic acid molecule of the invention will have at least about 85%, 90%, 91%, 92%, 93%, 94%, 95%, 96%, 97%, 98%, 99% or more sequence identity to that particular nucleic acid molecule as determined by sequence alignment programs and parameters.

"Variant protein" in the present invention is intended to mean a protein derived from the reference protein by deletion or addition of one or more amino acids at one or more internal sites in the protoporphyrinogen oxidase and/or substitution of one or more amino acids at one or more sites in the protoporphyrinogen oxidase. Variant proteins encompassed by the present invention are biologically active, that is they continue to possess the desired biological activity of the protoporphyrinogen oxidase of the invention, that is, protoporphyrinogen oxidase activity and/or herbicide tolerance as described herein. Such variants may result from, for example, genetic polymorphism or from human manipulation. Biologically active variants of a protoporphyrinogen oxidase of the invention will have at least about 88%, 90%, 91%, 92%, 93%, 94%, 95%, 96%, 97%, 98%, 99% or more sequence identity across the entirety of the amino acid sequence for the protoporphyrinogen oxidase as determined by sequence alignment programs and parameters. A biologically active variant of a protein of the invention may differ from that protein by as few as 1-15 amino acid residues, as few as 1-10, such as 6-10, as few as 5, as few as 4, 3, 2, or even 1, amino acid residue.

In certain examples, the nucleotide sequences encoding protoporphyrinogen oxidases of the present invention, or the protoporphyrinogen oxidases of the variants thereof that retain protoporphyrinogen oxidase activity, can be stacked with any combination of nucleotide sequences of interest in order to create plants with a desired trait. The term "trait" refers to the phenotype derived from a particular sequence or groups of sequences. For example, the amino acids/polynucleotides encoding a protoporphyrinogen oxidase of the invention or variant thereof that retains protoporphyrinogen oxidase activity may be stacked with any other nucleotides encoding polypeptides that confer a desirable trait, including but not limited to resistance to diseases, insects, and herbicides, tolerance to heat and drought, reduced time to crop maturity, improved industrial processing, such as for the conversion of starch or biomass to fermentable sugars, and improved agronomic quality, such as high oil content and high protein content.

It is well-known for a person skilled in the art that the benefits of a combination of two or more modes of action in improving the spectrum of weeds controlled and/or the control of naturally more tolerant species or resistant weed species, can also be extended to chemicals for which herbicide tolerance was enabled in crops through artificial methods (either transgenically or non-transgenically) beyond PPO tolerant crops. In fact, the traits encoding the following resistances can be superposed alone or in multiple combinations to provide the ability to effectively control or prevent weed shifts to herbicides: glyphosate resistance (such as EPSPS, GOX, and GAT from resistant plants or bacteria), glufosinate resistance (such as PAT and Bar), herbicide resistance to acetolactate synthase (ALS) inhibitors (such as imidazolinones, sulfonyl urea, triazole pyrimidines, sulfonated aniline, pyrimidinyl thiobenzoic acids and other chemicals resistant genes, e.g., AHAS, Csrl, and SurA), phenoxyauxin herbicide resistance (such as aryloxyalkanoate dioxygenase-12 (AAD-12)), dicamba herbicide resistance (such as dicamba monooxygenase (DMO)), bromoxynil resistance (such as Bxn), phytoene desaturase (PDS) inhibitor resistance, herbicide resistance to photosystem II inhibitors (such as psbA), herbicide resistance to photosystem I inhibitors, herbicide resistance to 4- hydroxyphenylpyruvate dioxygenase (HPPD) inhibitors (such as PPO-1), phenylurea herbicide resistance (such as CYP76B 1), and dichloromethoxybenzoic acid degrading enzymes.

Glyphosate is widely used, as it controls a very broad spectrum of broad-leaf and gramineous weed species. However, repeat use of glyphosate in glyphosate-tolerant crop and non-crop applications has (and will continue to) selected for weed shifts to naturally more tolerant species or glyphosate resistant biotypes. Most herbicide resistance management programs suggest using an effective dose of tank-mixed herbicide partners as a means for delaying the emergence of resistant weeds, wherein the herbicide partners provide control for the same species, but have different modes of action. Superposing the gene encoding the protoporphyrinogen oxidase of the present invention with a glyphosate tolerance trait (and/or other herbicide tolerance traits) can achieve the control of glyphosate resistant weed species (broad-leaf weed species controlled by one or more PPO-inhibitor herbicides) in glyphosate tolerant crops by enabling the selective use of glyphosate and PPO-inhibitor herbicides (such as oxyfluorfen, saflufenacil and flumioxazin) in the same crop. The applications of these herbicides can be performed simultaneously in a tank mixture containing two or more herbicides with different modes of action, or can be performed alone in a single herbicide composition in sequential applications (e.g., before planting, or before or after emergence) (with the interval time of applications ranging from 2 hours to 3 months); or alternatively, the applications of these herbicides can be performed by using a combination of any number of herbicides representative of each applicable compound category at any time (from 7 months after planting a crop to the time when the crop is harvested (or the pre-harvest interval for a single herbicide, wherein the shortest is taken)).

The flexibility in controlling broad-leaf weeds is very important, in terms of the application time, application amount of single herbicide, and abilities to control the stubborn or resistant weeds. The application range of glyphosate superposed with a glyphosate resistant gene/gene encoding the protoporphyrinogen oxidase of the present invention in crops can be from 250 to 2500 g ae/ha. The application range of PPO-inhibitor herbicides (one or more) can be from 10 to 1000g ai/ha. The optimal combination of time for these applications depends on the specific conditions, species and environments.

An herbicide formulation (e.g., an ester, acid or salt-formulation, or soluble concentrate, emulsifiable concentrate or soluble liquid) and a tank mix additive (e.g., an adjuvant or compatilizer) can significantly affect the weed control of a given herbicide or a combination of one or more herbicides. Any chemical combination of any of the foregoing herbicides is within the scope of the present invention.

In addition, the gene encoding the protoporphyrinogen oxidase of the present invention alone or being stacked with other characteristics of herbicide resistant crops can be stacked with one or more other input traits (for example, insect resistance, fungal resistance or stress tolerance, etc.) or output traits (for example, increased yield, improved oil amount, increased fiber quality, etc.). Therefore, the present invention can be used to provide complete agricultural solutions for improving the qualities of crops with the abilities for flexibly and economically controlling any number of agriculture pests.

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system.

The gene encoding the protoporphyrinogen oxidase according to the present invention has higher tolerance to PPO-inhibitor herbicides, which is an important basis for herbicide tolerant crops and selectable marker trait opportunities.

The term "expression cassette" as used herein means a nucleic acid molecule capable of directing expression of a particular polynucleotide sequence in an appropriate host cell, comprising a promoter effectively linked to the polynucleotide sequence of interest (i.e., a polynucleotide encoding a protoporphyrinogen oxidase of the present invention or variant protein thereof that retains protoporphyrinogen oxidase activity, alone or in combination with one or more additional nucleic acid molecules encoding polypeptides that confer desirable traits) which is effectively linked to termination signals. The coding region usually codes for a protein of interest but may also code for a functional RNA of interest, for example antisense RNA or a non-translated RNA, in the sense or antisense direction. The expression cassette comprising the polynucleotide sequence of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into new host cell by a transformation event. The expression of the polynucleotide sequence in the expression cassette may be under the control of a constitutive promoter or of an inducible promoter that initiates transcription only when the host cell is exposed to some particular external stimulus. Additionally, the promoter can also be specific to a particular tissue or organ or stage of development.

The present invention encompasses the transformation of plants with expression cassettes capable of expressing a polynucleotide of interest (i.e., a polynucleotide encoding a protoporphyrinogen oxidase of the present invention or variant protein thereof that retains protoporphyrinogen oxidase activity, alone or in combination with one or more additional nucleic acid molecules encoding polypeptides that confer desirable traits). The expression cassette will include in the 5'-3' direction of transcription, a transcriptional and translational initiation region (i.e., a promoter) and a polynucleotide open reading frame. The expression cassette may optionally comprise a transcriptional and translational termination region (i.e., termination region) functional in plants. In some embodiments, the expression cassette comprises a selectable marker gene to allow for selection for stable transformants. Expression constructs of the invention may also comprise a leader sequence and/or a sequence allowing for inducible expression of the polynucleotide of interest.

The regulatory sequences of the expression construct are effectively linked to the polynucleotide of interest. The regulatory sequence in the present invention includes, but is not limited to, a promoter, a transit peptide, a terminator, an enhancer, a leader sequence, an intron and other regulatory sequences operably linked to the gene encoding the protoporphyrinogen oxidase.

The promoter is a plant expressible promoter. The "plant expressible promoter" refers to a promoter that ensures the expression of the coding sequence linked thereto in a plant cell. The plant expressible promoter can be a constitutive promoter. Examples of the promoters directing the constitutive expression in plants include, but are not limited to, a 35S promoter derived from a cauliflower mosaic virus, maize Ubi promoters, rice GOS2 gene promoters, and the like. Alternatively, the plant expressible promoter can be a tissue specific promoter, i.e. the promoter directs the expression of an coding sequence in several tissues, such as green tissues, at a level higher than in other tissues of the plant (which can be measured through conventional RNA trials), such as a PEP carboxylase promoter. Alternatively, the plant expressible promoter can be a wound-inducible promoter. The wound-inducible promoter or a promoter directing a wound-induced expression pattern means that when a plant suffers from a wound caused by a mechanical factor or the gnawing of insects, the expression of the coding sequence under the regulation of the promoter is significantly improved compared to normal growth conditions. Examples of the wound-inducible promoters include, but are not limited to, promoters of potato and tomato protease inhibitor genes (pin I and pin II) and a maize protease inhibitor gene (MPI).

The transit peptide (also known as secretion signal sequence or targeting sequence) directs a transgenic product to a specific organelle or cell compartment. For a receptor protein, the transit peptide may be heterologous, for example, targeting the chloroplast using a sequence encoding the chloroplast transit peptide, or targeting the endoplasmic reticulum using a 'KDEL' retention sequence, or targeting the vacuole using CTPP of a barley phytolectin gene.

The leader sequence includes, but is not limited to, a small RNA virus leader sequence, such as an EMCV leader sequence (a 5' non-coding region of encephlomyocarditis virus); a potato virus Y group leader sequence, such as a MDMV (Maize Dwarf Mosaic Virus) leader sequence; human immunoglobulin heavy chain binding protein (BiP); an untranslated leader sequence of the coat protein mRNA of alfalfa mosaic virus (AMV RNA4); and a tobacco mosaic virus (TMV) leader sequence.

The enhancer includes, but is not limited to, a cauliflower mosaic virus (CaMV) enhancer, figwort mosaic virus (FMV) enhancer, carnation etched ring virus (CERV) enhancer, cassava vein mosaic virus (CsVMV) enhancer, mirabilis mosaic virus (MMV) enhancer, cestrum yellow leaf curling virus (CmYLCV) enhancer, cotton leaf curl Multan virus (CLCuMV) enhancer, commelina yellow mottle virus (CoYMV) enhancer and peanut chlorotic streak caulimovirus (PCLSV) enhancer.

For use in a monocotyledonous plant, the intron includes, but is not limited to, a maize hsp70 intron, maize ubiquitin intron, Adh intron 1, sucrose synthase intron or rice Act1 intron. For use in a dicotyledonous plant, the intron includes, but is not limited to, a CAT-1 intron, pKANNIBAL intron, PIV2 intron and "super ubiquitin" intron.

The terminator can be a suitable polyadenylation signal sequence that functions in a plant, including, but not limited to, a polyadenylation signal sequence derived from the Agrobacterium tumefaciens nopaline synthetase (NOS) gene, a polyadenylation signal sequence derived from the protease inhibitor II (pinII) gene, a polyadenylation signal sequence derived from the pea ssRUBISCO E9 gene and a polyadenylation signal sequence derived from the α-tubulin gene.

The "effectively linking" in the present invention indicates the binding of nucleic acid sequences that enables one of the sequences to provide a function required for the sequence linked thereto. The "effectively linking" in the present invention can link a promoter to a sequence of interest, so that the transcription of the sequence of interest is controlled and regulated by the promoter. When a sequence of interest encodes a protein and the expression of the protein is desired, "effectively linking" means that: a promoter is linked to the sequence in such a manner that the resulting transcript is efficiently translated. If the linking of a promoter to a coding sequence is a transcript fusion and expression of the encoded protein is to be achieved, such linking is created that the first translation initiation codon in the resulting transcript is the initiation codon in the coding sequence. Alternatively, if the linking of a promoter to a coding sequence is a translation fusion and expression of the encoded protein is to be achieved, such a linking is created that the first translation initiation codon contained in the 5' untranslated sequence is linked to the promoter in such a manner that the relationship of the resulting translation product with the translation open reading frame encoding the desired protein is an in-frame. Nucleic acid sequences that can be "effectively linked" include, but are not limited to: sequences providing gene expression functions (i.e., gene expression elements, such as promoters, 5' untranslated regions, introns, protein coding regions, 3' untranslated regions, polyadenylation sites and/or transcription terminators), sequences providing DNA transfer and/or integration functions (i.e., T-DNA boundary sequences, site-specific recombinase recognition sites and integrase recognition sites), sequences providing selective functions (i.e., antibiotic resistance markers and biosynthesis genes), sequences providing marker scoring functions, sequences assisting in sequence manipulation in vitro or in vivo (i.e., polylinker sequences and site-specific recombination sequences), and sequences providing replication functions (i.e., the bacterial origins of replication, autonomously replicating sequences and centromeric sequences).

The genome of a plant, plant tissue or plant cell in the present invention refers to any genetic material within the plant, plant tissue or plant cell, and includes nuclear, plastid and mitochondrial genomes.

As used herein, the term "plant part" or "plant tissue" includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like.

The mutant PPO protein of the present invention can be applied to various types of plants. The dicotyledonous plant includes, but is not limited to, alfalfa, beans, cauliflowers, cabbages, carrots, celery, cotton, cucumbers, eggplants, lettuces, melon, peas, peppers, zucchinis, radishes, oilseed rape, spinach, soybeans, pumpkins, tomatoes, *Arabidopsis thaliana,* peanuts or watermelons; preferably, the dicotyledonous plant refers to cucumbers, soybeans, *Arabidopsis thaliana,* tobacco, cotton or oilseed rape. The monocotyledonous plant includes, but is not limited to, maize, rice, sorghum, wheat, barley, rye, millet, sugar cane, oats or turfgrass. Preferably, the monocotyledonous plant refers to maize, rice, sorghum, wheat, barley, millet, sugar cane or oats.

As used herein, the term "plant transformation" means that once an herbicide resistant or tolerant nucleic acid molecules encoding the protoporphyrinogen oxidase of the present invention, alone or in combination with one or more additional nucleic acid molecules encoding polypeptides that confer desirable traits, has been cloned into an expression system, it is transformed into a plant cell. The receptors and target expression cassettes of the present invention can be introduced into the plant cell in a number of art-recognized ways. The term "introducing" in the context of a polynucleotide, for example, a nucleotide construct of interest, is intended to mean presenting to the plant the polynucleotide in such a manner that the polynucleotide gains access to the interior of a cell of the plant. Where more than one polynucleotide is to be introduced, these polynucleotides can be assembled as part of a single nucleotide construct, or as separate nucleotide constructs, and can be located on the same or different transformation vectors. Accordingly, these polynucleotides can be introduced into the host cell of interest in a single transformation event, in separate transformation events, or, for example, in plants, as part of a breeding protocol. The methods of the invention do not depend on a particular method for introducing one or more polynucleotides into a plant, only that the polynucleotide(s) gains access to the interior of at least one cell of the plant. Methods for introducing one or more polynucleotides into plants are known in the art including, but not limited to, transient transformation methods, stable transformation methods, and virus-mediated methods or genome-editing techniques.

The term "stable transformation" means that an exogenous gene is introduced into the genome of the plant and stably integrates into the plant or its genome of any successive generations, so that the exogenous gene is stably inherited.

The term "transient transformation" means that a nucleic acid molecule or protein is introduced into the plant cell to execute the function, but does not integrate into the genome of the plant, so that an exogenous gene cannot be stably inherited.

The term "genome-editing technique" refers to the techniques used to modify the genome that are capable of precisely manipulating the genomic sequences to realize operations such as site-directed gene mutations, insertions and deletions. At present, the genome-editing techniques mainly include homing endonucleases (HEs), Zinc-finger nucleases (ZFNs), transcription activator-like effector nucleases (TALENs), and Clustered regulatory interspaced short palindromic repeat (CRISPR) technologies.

Numerous transformation vectors available for plant transformation are known to those of ordinary skills in the art, and the genes pertinent to this invention can be used in conjunction with any such vectors. The selection of vector will depend upon the preferred transformation technique and the target species for transformation. For certain target species, different antibiotic or herbicide selection markers may be preferred. Selection markers used routinely in transformation include the nptII gene (which was published by Bevan et al., Nature 304:184-187 (1983)), which confers resistance to kanamycin and related antibiotics or herbicides; the pat and bar genes, which confer resistance to the herbicide glufosinate (also called phosphinothricin; see White et al., Nucl. Acids Res 18: 1062 (1990), Spencer et al. Theon. Appl. Genet. 79: 625-631 (1990) and U.S. Pat. Nos. 5,561,236 and 5,276,268); the hph gene, which confers resistance to the antibiotic hygromycin (Blochinger & Diggelmann, Mol. Cell. Biol. 4: 2929-2931); the dhfr gene, which confers resistance to methatrexate (Bourouis et al., EMBO J. 2(7): 1099-1104 (1983)); the EPSPS gene, which confers resistance to glyphosate (U.S. Pat. Nos. 4,940,935 and 5,188,642); the glyphosate N-acetyltransferase (GAT) gene, which also confers resistance to glyphosate (Castle et al. (2004) Science, 304:1151-1154; U.S. Patent App. Pub. Nos. 20070004912, 20050246798, and 20050060767); and the mannose-6-phosphate isomerase gene, which provides the ability to metabolize mannose (U.S. Pat. Nos. 5,767,378 and 5,994,629).

Methods for regeneration of plants are also well-known in the art. For example, Ti plasmid vectors have been utilized for the delivery of foreign DNA, as well as direct DNA uptake, liposomes, electroporation, microinjection, and microprojectiles.

In the present invention, weeds refer to plants competing with the cultivated transgenic plants in the farmland.

The term "control" and/or "prevention" in the present invention refers to at least a direct application of (e.g., by spraying) an effective dose of an PPO-inhibitor herbicide to the farmland, so as to minimize weed development and/or stop weed growth. At the same time, the cultivated transgenic plants should be morphologically normal and can be cultivated under conventional methods for product consumption and/or generation; and preferably, compared to non-transgenic wild-type plants, the cultivated plants have reduced plant damage and/or an increased plant yield. The reduced plant damage includes, but is not limited to, an improved stem resistance and/or an increased grain weight, etc. The "control" and/or "prevention" effect of the protoporphyrinogen oxidase on weeds can exist independently, and will not be diminished and/or lost due to the presence of other substances that can "control" and/or "prevent" the weeds. Specifically, if any tissue of a transgenic plant (containing the gene encoding the protoporphyrinogen oxidase of the present invention) has and/or produces the protoporphyrinogen oxidase and/or another substance that can control weeds simultaneously and/or separately, then the presence of the another substance will neither affect the "control" and/or "prevention" effect of the protoporphyrinogen oxidase on the weeds, nor result in the "control" and/or "prevention" effect being completely and/or partially achieved by the another substance, regardless of the protoporphyrinogen oxidase.

The "plant propagule" in the present invention includes, but is not limited to, plant sexual propagules and plant vegetative propagules. The plant sexual propagules include, but are not limited to, plant seeds; and the plant vegetative propagules refer to vegetative organs or a specific tissue of a plant which can generate a new plant under *ex vivo* conditions. The vegetative organs or the specific tissue include, but are not limited to, roots, stems and leaves, for example: plants with roots as the vegetative propagules including strawberries, sweet potatoes and the like; plants with stems as the vegetative propagules including sugar cane, potatoes (tubers) and the like; and plants with leaves as the vegetative propagules including aloe, begonias and the like.

The present invention may confer a new herbicide resistance trait to a plant, and no adverse effects on the phenotypes (including yields) are observed. The plant in the present invention can tolerate, e.g., 2×, 3×, 4× or 5× the general application level of at least one herbicide tested. The improvement of these levels of tolerance is within the scope of the present invention. For example, foreseeable optimization and further development can be performed on various techniques known in the art, in order to increase the expression of a given gene.

The present invention provides use of a protoporphyrinogen oxidase, having the following advantages:
1. Wide tolerance to herbicide. The present invention first discloses that protoporphyrinogen oxidase PPO1-PPO14 can show higher tolerance to PPO inhibitor herbicides, thereby having wide application prospect in plants.
2. Strong tolerance to herbicide. The protoporphyrinogen oxidase PPO1-PPO14 disclosed by the present invention has strong tolerance to PPO inhibitor herbicides, and almost all of the protoporphyrinogen oxidase PPO1-PPO14 show high resistance to four-fold field concentration of oxyfluorfen, saflufenacil and flumioxazin, and two-fold field concentration of sulfentrazone.
3. Little influence on yield. The tolerance of plants to herbicides has a direct correlation with the yield of plants. High-resistance plants will not be affected by herbicides, which will not affect the yield of plants. However, the yield of middle and low-resistance plants will be much lower than that of high-resistance plants.

The technical solution of the present invention is further illustrated in details through the drawings and examples below.

### BRIEF DESCRIPTION OF THE DRAWINGS

FIG. 1 is a schematic structural diagram of a recombinant expression vector DBN11375containing the PPO1A nucleotide sequence for *Arabidopsis thaliana* according to the present invention;
FIG. 2 is a schematic structural diagram of a control recombinant expression vector DBN12337N according to the present invention;
FIG. 3 is a schematic structural diagram of a recombinant expression vector DBN11375containing the PPO1B nucleotide sequence for *maize* according to the present invention;
FIG. 4 is a schematic structural diagram of a control recombinant expression vector DBN12354N according to the present invention.

### DETAILED DESCRIPTION OF THE INVENTION

The technical solutions regarding use of the protoporphyrinogen oxidases of the present invention will be further illustrated by the following examples.

### Example 1 Acquisition and verification of transgenic Arabidopsis thaliana plants

### 1. Acquisition of genes encoding protoporphyrinogen oxidases

The amino acid sequences of the microbial protoporphyrinogen oxidases PPO1, PPO2, PPO3, PPO4, PPOS, PPO6, PPO7, PPO8, PPO9, PPO10, PPO11, PPO12, PPO13 and PPO14 are set forth as SEQ ID NO:1-14 in the SEQUENCE LISTING; the PPO1-PPO14 nucleotide sequences encoding the corresponding protoporphyrinogen oxidases PPO1-PPO14 are set forth as SEQ ID NO:15-28 in the SEQUENCE LISTING; the PPO1A-PPO14A nucleotide sequences encoding the corresponding protoporphyrinogen oxidases PPO1-PPO14, which were obtained based on the *Arabidopsis thaliana*/soybean common codon usage bias, are set forth as SEQ ID NO:29-42 in the SEQUENCE LISTING; and the PPO1B, PPO6B and PPO12B nucleotide sequences encoding the corresponding protoporphyrinogen oxidases PPO1, PPO6 and PPO12, which were obtained based on the maize common codon usage bias, are set forth as SEQ ID NO:62-64 in the SEQUENCE LISTING.

The amino acid sequence of the *Escherichia coli* protoporphyrinogen oxidase PPO-EC is set forth as SEQ ID NO:43 in the SEQUENCE LISTING; the PPO-EC nucleotide sequence encoding the corresponding *Escherichia coli* protoporphyrinogen oxidase PPO-EC is set forth as SEQ ID NO:44 in the SEQUENCE LISTING; and the PPO-ECA nucleotide sequence encoding the corresponding *Escherichia coli* protoporphyrinogen oxidase PPO-EC, which was obtained based on the *Arabidopsis thaliana*/soybean common codon usage bias, is set forth as SEQ ID NO:45 in the SEQUENCE LISTING.

The amino acid sequence of the *Arabidopsis* protoporphyrinogen oxidase PPO-AT is set forth as SEQ ID NO:46 in the SEQUENCE LISTING; the PPO-AT nucleotide sequence encoding the corresponding *Arabidopsis* protoporphyrinogen oxidase PPO-AT is set forth as SEQ ID NO:47 in the SEQUENCE LISTING; and the PPO-ATA nucleotide sequence encoding the corresponding *Arabidopsis* protoporphyrinogen oxidase PPO-AT, which was obtained based on the *Arabidopsis thaliana*/soybean common codon usage bias, is set forth as SEQ ID NO:48 in the SEQUENCE LISTING.

The amino acid sequence of the *Arsenophonus* protoporphyrinogen oxidase PPO-AP is set forth as SEQ ID NO:65 in the SEQUENCE LISTING; the PPO-AP nucleotide sequence encoding the corresponding *Arsenophonus* protoporphyrinogen oxidase PPO-AP is set forth as SEQ ID NO:66 in the SEQUENCE LISTING; the PPO-APA nucleotide sequence encoding the corresponding *Arsenophonus* protoporphyrinogen oxidase PPO-AP, which was obtained based on the *Arabidopsis thaliana*/soybean common codon usage bias, is set forth as SEQ ID NO:67 in the SEQUENCE LISTING; and the PPO-APB nucleotide sequence encoding the corresponding *Arsenophonus* protoporphyrinogen oxidase PPO-AP, which was obtained based on the maize common codon usage bias, is set forth as SEQ ID NO:68 in the SEQUENCE LISTING.

### 2. Synthesis of the aforementioned nucleotide sequences

The 5' and 3' ends of the PPO1A-PPO14A nucleotide sequences, PPO-ECA nucleotide sequence, PPO-ATA nucleotide sequence, and PPO-APA nucleotide sequence (SEQ ID NO:29-42, SEQ ID NO:45, SEQ ID NO:48, and SEQ ID NO:67) were respectively linked to a universal adapter primer 1:
Universal adapter primer 1 for the 5' end: 5'-taagaaggagatatacatatg-3', as set forth in SEQ ID NO:49 in the SEQUENCE LISTING;
Universal adapter primer 1 for the 3' end: 5'-gtggtggtggtggtgctcgag-3', as set forth in SEQ ID NO:50 in the SEQUENCE LISTING.

### 3. Construction of recombinant expression vectors containing the PPO1A-PPO14A nucleotide sequences, PPO-ECA nucleotide sequence, PPO-ATA nucleotide sequence, and PPO-APA nucleotide sequence for Arabidopsis thaliana

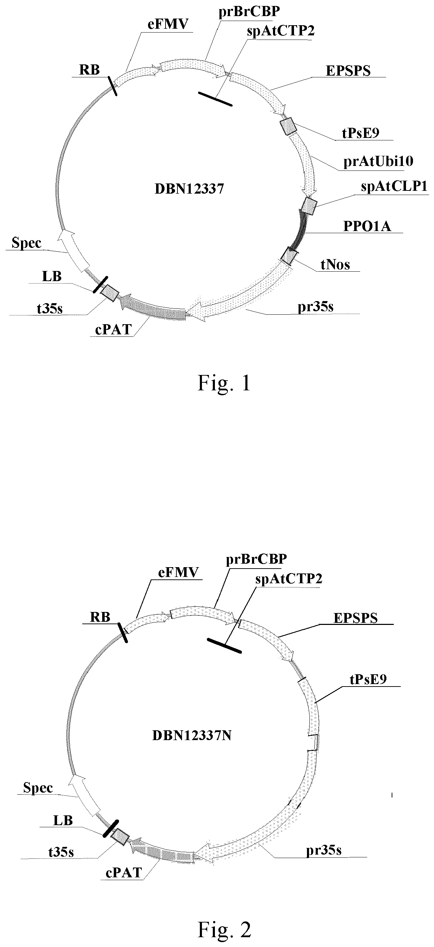

A plant expression vector DBNBC-01 was subjected to double digestion using restriction enzymes *Spe I* and *Asc I* to linearize the plant expression vector. The digestion product was purified to obtain the linearized DBNBC-01 expression vector backbone (vector backbone: pCAMBIA2301 (which is available from CAMBIA)) which then underwent a recombination reaction with the PP01A nucleotide sequence (SEQ ID NO:29) linked to the universal adapter primer 1, according to the procedure of Takara In-Fusion products seamless connection kit (Clontech, CA, USA, CAT: 121416) instructions, to construct a recombinant expression vector DBN12337 with the schematic structure as shown in FIG. 1 (Spec: spectinomycin gene; RB: right border; eFMV: 34S enhancer of Figwort mosaic virus (SEQ ID NO: 51); prBrCBP: promoter of oilseed rape eukaryotic elongation factor gene 1α (Tsf1) (SEQ ID NO: 52); spAtCTP2: *Arabidopsis thaliana* chloroplast transit peptide (SEQ ID NO: 53); EPSPS: 5-enolpyruvylshikimate-3-phosphate synthase gene (SEQ ID NO: 54); tPsE9: terminator of a pea RbcS gene (SEQ ID NO: 55); prAtUbi10: promoter of an *Arabidopsis thaliana* Ubiquitin 10 gene (SEQ ID NO: 56); spAtCLP1: *Arabidopsis thaliana* albino or pale-green chloroplast transit peptide (SEQ ID NO:57); PPO1A: PPO1A nucleotide sequence (SEQ ID NO:29); tNos: terminator of a nopaline synthase gene (SEQ ID NO: 58); pr35S: the cauliflower mosaic virus 35S promoter (SEQ ID NO: 59); cPAT: phosphinothricin acetyltransferase gene (SEQ ID NO: 60); t35S: cauliflower mosaic virus 35S terminator (SEQ ID NO: 61); LB: left border).

*Escherichia coli* T₁ competent cells were transformed with the recombinant expression vector DBN12337 by using a heat shock method under the following heat shock conditions: 50 µL of *Escherichia coli* T₁ competent cells and 10 µL of plasmid DNA (recombinant expression vector DBN12337) were water-bathed at 42°C for 30 seconds, shake cultured at 37°C for 1 hour (using a shaker at a rotation speed of 100 rpm for shaking), and then cultured under the condition of a temperature of 37°C on the LB solid plate (10 g/L of tryptone, 5 g/L of yeast extract, 10 g/L of NaCl, and 15 mg/L of agar; adjusted to a pH of 7.5 with NaOH) containing 50 mg/L of spectinomycin for 12 hours; white bacterial colonies were picked out, and cultured under the condition of a temperature of 37°C overnight in an LB liquid culture medium (10 g/L of tryptone, 5 g/L of yeast extract, 10 g/L of NaCl, and 50 mg/L of spectinomycin; adjusted to a pH of 7.5 with NaOH). The plasmids in the cells were extracted through an alkaline method: the bacteria solution was centrifuged at a rotation speed of 12,000 rpm for 1 min, the supernatant was removed, and the precipitated thalli were suspended with 100 µL of ice pre-cooled solution I (25 mM Tris-HCl, 10 mM EDTA (ethylenediaminetetraacetic acid), and 50 mM glucose, with a pH of 8.0); 200 µL of newly prepared solution II (0.2M NaOH, 1% SDS (sodium dodecyl sulfate)) was added, mixed by inverting the tube 4 times, and placed on ice for 3-5 min; 150 µL of ice-cold solution III (3 M potassium acetate, 5 M acetic acid) was added, mixed uniformly immediately and placed on ice for 5-10 min; the mixture was centrifuged under the conditions of a temperature of 4°C and a rotation speed of 12,000 rpm for 5 min, 2-fold volumes of anhydrous ethanol was added to the supernatant, mixed uniformly and placed at room temperature for 5 min; the mixture was centrifuged under the conditions of a temperature of 4°C and a rotation speed of 12,000 rpm for 5 min, the supernatant was discarded, and the precipitate was washed with ethanol at a concentration of 70% (V/V) and then was air dried; 30 µL of TE (10 mM Tris-HCl, and 1 mM EDTA, with a pH of 8.0) containing RNase (20 µg/mL) was added to dissolve the precipitate; the obtained product was water bathed at a temperature of 37°C for 30 min to digest the RNA; and stored at a temperature of -20°C for use. The extracted plasmids were identified by sequencing. The results showed that the nucleotide sequence between the *SpeI* and *AscI* sites in the recombinant expression vector DBN12337 was the one as set forth in SEQ ID NO:29 in the SEQUENCE LISTING, i.e., the PPO1A nucleotide sequence.

According to the above method for constructing recombinant expression vector DBN12337, the PPO2A-PPO14A nucleotide sequences, PPO-ECA nucleotide sequence, PPO-ATA nucleotide sequence, and PPO-APA nucleotide sequence which were linked to the universal adapter primer 1, were respectively subjected to a recombination reaction with the linearized DBNBC-01 expression vector backbone, to construct the recombinant expression vectors DBN12338 to DBN12353 in sequence. Sequencing verified that the above-mentioned nucleotide sequences were correctly inserted in the recombinant expression vectors DBN12338 to DBN12353.

According to the method for constructing the recombinant expression vector DBN12337 as described above, the recombinant expression vector DBN12337N was constructed as the control, and its structure was shown in FIG. 2 (Spec: the spectinomycin gene; RB: right border; eFMV: 34S enhancer of Figwort mosaic virus (SEQ ID NO: 51); prBrCBP: promoter of oilseed rape eukaryotic elongation factor gene 1α (Tsf1) (SEQ ID NO: 52); spAtCTP2: *Arabidopsis thaliana* chloroplast transit peptide (SEQ ID NO:53); EPSPS: 5-enolpyruvylshikimate-3-phosphate synthase gene (SEQ ID NO:54); tPsE9: terminator of a pea RbcS gene (SEQ ID NO: 55); pr35S: the cauliflower mosaic virus 35S promoter (SEQ ID NO:59); cPAT: phosphinothricin acetyltransferase gene (SEQ ID NO:60); t35S: cauliflower mosaic virus 35S terminator (SEQ ID NO: 61); LB: left border).

### 4. Transformation of Agrobacterium with the recombinant expression vectors for Arabidopsis thaliana

The recombinant expression vectors DBN12337 to DBN12350, DBN12352, DBN12353, and the above-mentioned control recombinant expression vector DBN12337N which had been constructed correctly were respectively transformed into Agrobacterium GV3101 using a liquid nitrogen method, under the following transformation conditions: 100 µL of Agrobacterium GV3101 and 3 µL of plasmid DNA (recombinant expression vectors DBN12337 to DBN12350, DBN12352, DBN12353 and DBN12337N) were placed in liquid nitrogen for 10 minutes, and bathed in warm water at 37°C for 10 min; the transformed Agrobacterium GV3101 was inoculated into an LB tube, cultured under the conditions of a temperature of 28°C and a rotation speed of 200 rpm for 2 hours, and spread on the LB solid plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN12337 to DBN12350, DBN12352, DBN12353, and DBN12337N were completely correct.

### 5. Acquisition of transgenic Arabidopsis thaliana plants

Seeds of wild-type *Arabidopsis thaliana* were suspended in a 0.1% (w/v) agarose solution. The suspended seeds were stored at 4°C for 2 days to fulfill the need for dormancy, in order to ensure synchronous seed germination. Vermiculite was mixed with horse manure soil, the mixture was sub-irrigated with water to wet, and the soil mixture was allowed to drain the water away for 24 hours. The pretreated seeds were sowed in the soil mixture and covered with a moisturizing cover for 7 days. The seeds were germinated and the plants were cultivated in a greenhouse under long sunlight conditions (16-hour light/8-hour dark) at a constant temperature (22°C) and a constant humidity (40-50%), with a light intensity of 120-150 µmol/m²s⁻¹. The plants were initially irrigated with Hoagland's nutrient solution and then with deionized water, thus keeping the soil moist, but not water penetrated.

*Arabidopsis thaliana* was transformed using the flower soaking method. One or more 15-30 mL pre-cultures of a LB culture solution containing spectinomycin (50 mg/L) and rifampicin (10 mg/L) were inoculated with the picked Agrobacterium colonies. The pre-cultures were incubated at a temperature of 28° C and a rotation speed of 220 rpm with shaking at a constant speed overnight. Each pre-culture was used to inoculate two 500 mL cultures of the YEP culture solution containing spectinomycin (50 mg/L) and rifampicin (10 mg/L), and the cultures were incubated at 28°C with continuous shaking overnight. Centrifugation at a rotation speed of about 4,000 rpm was carried out at room temperature for 20 minutes to precipitate cells, and the resulting supernatant was discarded. The cell precipitate was gently re-suspended in 500 mL of an osmotic medium which contained 1/2×MS salt/B5 vitamin, 10% (w/v) sucrose, 0.044 µM of benzylaminopurine (10 µL/L (1 mg/mL stock solution in DMSO)) and 300 µL/L of Silvet L-77. About 1-month-old *Arabidopsis thaliana* plants were soaked in an osmotic culture medium which contained re-suspended cells for 15 seconds to ensure immersion of the latest inflorescence. Then, the *Arabidopsis thaliana* plants were reclined laterally and covered and they were kept wet in dark for 24 hours. The *Arabidopsis thaliana* plants were normally cultivated with a photoperiod of 16 hours of light/8 hours of darkness at 22°C. Seeds were harvested after about 4 weeks.

The newly harvested (PPO1A-PPO14A nucleotide sequences, PPO-ATA nucleotide sequence, PPO-APA nucleotide sequence and control vector DBN12337N) T₁ seeds were dried at room temperature for 7 days. The seeds were sowed in 26.5 cm ×51 cm germination disks, and 200 mg of T₁ seeds (about 10,000 seeds) were accepted per disk, wherein the seeds had been previously suspended in distilled water and stored at 4°C for 2 days to fulfill the need for dormancy, in order to ensure synchronous seed germination.

Vermiculite was mixed with horse manure soil, the mixture was sub-irrigated with water to wet, and water was drained by gravity. The pretreated seeds were sowed evenly in the soil mixture using a pipette, and covered with a moisturizing cover for 4-5 days. The cover was removed 1 day before the post-emergence spraying application of glufosinate (used to select the co-transformed PAT gene) for the selection of initial transformant.

The T₁ plants were sprayed with a 0.2% solution of a Liberty herbicide (200 g ai/L of glufosinate) by a DeVilbiss compressed air nozzle at a spray volume of 10 mL/disk (703 L/ha) 7 days after planting (DAP) and 11 DAP (the cotyledon stage and 2-4 leaf stage, respectively) to provide an effective amount of glufosinate of 280 g ai/ha per application. Surviving plants (actively growing plants) were identified 4-7 days after the final spraying, and transplanted to 7 cm×7 cm square pots prepared from horse manure soil and vermiculite (3-5 plants/disk). The transplanted plants were covered with a moisturizing cover for 3-4 days, and placed in a 22°C culture chamber or directly transferred into a greenhouse as described above. Then, the cover was removed, and at least 1 day before testing the ability of the PPO1A-PPO14A nucleotide sequences, PPO-ATA nucleotide sequence, PPO-APA nucleotide sequence, and the control vector to provide tolerance to PPO-inhibitor herbicides, the plants were planted in a greenhouse (22±5°C, 50±30% RH, 14 hours of light: 10 hours of darkness, a minimum of 500 µE/m²s⁻¹ natural + supplemental light).

### 6. Detection of the herbicide tolerance of the transgenic Arabidopsis thaliana plants

The transformed *Arabidopsis thaliana* T₁ plants were initially selected using glufosinate herbicide. The *Arabidopsis thaliana* T₁ plants (PPO1A) into which the PPO1A nucleotide sequence was introduced, the *Arabidopsis thaliana* T₁ plants (PPO2A) into which the PPO2A nucleotide sequence was introduced, the *Arabidopsis thaliana* T₁ plants (PPO3A) into which the PPO3A nucleotide sequence was introduced, the *Arabidopsis thaliana* T₁ plants (PPO4A) into which the PPO4A nucleotide sequence was introduced, the *Arabidopsis thaliana* T₁ plants (PPO5A) into which the PPO5A nucleotide sequence was introduced, the *Arabidopsis thaliana* T₁ plants (PPO6A) into which the PPO6A nucleotide sequence was introduced, the *Arabidopsis thaliana* T₁ plants (PPO7A) into which the PPO7A nucleotide sequence was introduced, the *Arabidopsis thaliana* T₁ plants (PPO8A) into which the PPO8A nucleotide sequence was introduced, the *Arabidopsis thaliana* T1 plants (PPO9A) into which the PPO9A nucleotide sequence was introduced, the *Arabidopsis thaliana* T₁ plants (PPO10A) into which the PPO10A nucleotide sequence was introduced, the *Arabidopsis thaliana* T₁ plants (PPO11A) into which the PPO11A nucleotide sequence was introduced, the *Arabidopsis thaliana* T₁ plants (PPO12A) into which the PPO12A nucleotide sequence was introduced, the *Arabidopsis thaliana* T₁ plants (PPO13A) into which the PPO13A nucleotide sequence was introduced, the *Arabidopsis thaliana* T₁ plants (PPO14A) into which the PPO14A nucleotide sequence was introduced, the *Arabidopsis thaliana* T₁ plants (PPO-ATA) into which the PPO-ATA nucleotide sequence was introduced, the *Arabidopsis thaliana* T₁ plants (PPO-APA) into which the PPO-APA nucleotide sequence was introduced, the *Arabidopsis thaliana* T₁ plants (control vector) into which the control vector was introduced, and the wild-type *Arabidopsis thaliana* plants (CK) (with 24 plants being included in each genotype) (18 days after sowing) were sprayed with oxyfluorfen at three concentrations (180 g ai/ha (one-fold field concentration, 1×), 720 g ai/ha (four-fold field concentration, 4×), and 0 g ai/ha (water, 0×)), saflufenacil at three concentrations (25 g ai/ha (one-fold field concentration, 1×), 100 g ai/ha (four-fold field concentration, 4×), and 0 g ai/ha (water, 0×)), flumioxazin at three concentrations (60 g ai/ha (one-fold field concentration, 1×), 240 g ai/ha (four-fold field concentration, 4×), and 0 g ai/ha (water, 0×), and sulfentrazone at three concentrations (450 g ai/ha (one-fold field concentration, 1 ×), 900 g ai/ha (two-fold field concentration, 2×), and 0 g ai/ha (water, 0×) to determine the tolerance of *Arabidopsis thaliana* to the herbicides. After 7 days of spraying (7 DAT), the damage level of each plant caused by the herbicide was evaluated according to the average of the damage levels (%) of plants (the average of the damage levels (%) of plants = the injured leaf area / the total leaf area × 100%), i.e., the grade of pesticide damage: grade 0 means that the growth status of plants is essentially consistent with those sprayed with the blank solvent (water); grade 1 means that the average of the damage levels of plants is less than 10%; grade 2 means that the average of the damage levels of plants is greater than 10%; and grade 3 means that the average of the damage levels of plants is 100%. The plants having a growth status falling within grades 0 and 1 are classified as highly resistant plants; those having a growth status falling within grade 2 are classified as poorly resistant plants; and those having a growth status falling within grade 3 are classified as non-resistant plants. The experimental results are shown in Tables 1-4.

**Table 1 Experimental results of the oxyfluorfen tolerance of Arabidopsis thaliana T₁ plants into which the PPO1A-PPO14A nucleotide sequences, PPO-APA nucleotide sequence and PPO-ATA nucleotide sequence were introduced**

| *Arabidopsis thaliana* genotype | Concentration (g ai/ha) | The grade of pesticide damage/plant | | | |
|---|---|---|---|---|---|
| | | 0 | 1 | 2 | 3 |
| CK | 0 | 24 | 0 | 0 | 0 |
| | 180 | 0 | 0 | 0 | 24 |
| | 720 | 0 | 0 | 0 | 24 |
| Control vector | 0 | 24 | 0 | 0 | 0 |
| | 180 | 0 | 0 | 0 | 24 |
| | 720 | 0 | 0 | 0 | 24 |
| PPO1A | 0 | 24 | 0 | 0 | 0 |
| | 180 | 24 | 0 | 0 | 0 |
| | 720 | 19 | 5 | 0 | 0 |
| PPO2A | 0 | 24 | 0 | 0 | 0 |
| | 180 | 24 | 0 | 0 | 0 |
| | 720 | 20 | 4 | 0 | 0 |
| PPO3A | 0 | 24 | 0 | 0 | 0 |
| | 180 | 24 | 0 | 0 | 0 |
| | 720 | 20 | 4 | 0 | 0 |
| PPO4A | 0 | 24 | 0 | 0 | 0 |
| | 180 | 24 | 0 | 0 | 0 |
| | 720 | 22 | 2 | 0 | 0 |
| PPO5A | 0 | 24 | 0 | 0 | 0 |
| | 180 | 24 | 0 | 0 | 0 |
| | 720 | 22 | 2 | 0 | 0 |
| PPO6A | 0 | 24 | 0 | 0 | 0 |
| | 180 | 24 | 0 | 0 | 0 |
| | 720 | 20 | 4 | 0 | 0 |
| PPO7A | 0 | 24 | 0 | 0 | 0 |
| | 180 | 24 | 0 | 0 | 0 |
| | 720 | 20 | 4 | 0 | 0 |
| PPO8A | 0 | 24 | 0 | 0 | 0 |
| | 180 | 24 | 0 | 0 | 0 |
| | 720 | 20 | 4 | 0 | 0 |
| PPO9A | 0 | 24 | 0 | 0 | 0 |
| | 180 | 24 | 0 | 0 | 0 |
| | 720 | 20 | 4 | 0 | 0 |
| PPO10A | 0 | 24 | 0 | 0 | 0 |
| | 180 | 24 | 0 | 0 | 0 |
| | 720 | 20 | 4 | 0 | 0 |
| PPO11A | 0 | 24 | 0 | 0 | 0 |
| | 180 | 24 | 0 | 0 | 0 |
| | 720 | 21 | 3 | 0 | 0 |
| PPO12A | 0 | 24 | 0 | 0 | 0 |
| | 180 | 24 | 0 | 0 | 0 |
| | 720 | 20 | 4 | 0 | 0 |
| PPO13A | 0 | 24 | 0 | 0 | 0 |
| | 180 | 24 | 0 | 0 | 0 |
| | 720 | 18 | 6 | 0 | 0 |
| PPO14A | 0 | 24 | 0 | 0 | 0 |
| | 180 | 24 | 0 | 0 | 0 |
| | 720 | 20 | 4 | 0 | 0 |
| PPO-APA | 0 | 24 | 0 | 0 | 0 |
| | 180 | 0 | 0 | 0 | 24 |
| | 720 | 0 | 0 | 0 | 24 |
| PPO-ATA | 0 | 24 | 0 | 0 | 0 |
| | 180 | 0 | 0 | 8 | 16 |
| | 720 | 0 | 0 | 0 | 24 |

For *Arabidopsis thaliana,* 180g ai/ha oxyfluorfen herbicide is an effective dose distinguishing sensitive plants from plants having an average level of resistance. The results of Table 1 show that compared with the control vector and CK, the genotypes PPO1A-PPO14A all exhibited high-resistant tolerance to oxyfluorfen at different concentrations, while the genotypes PPO-APA and PPO-ATA both exhibited basically no tolerance.

**Table 2 Experimental results of the saflufenacil tolerance of Arabidopsis thaliana T₁ plants into which the PPO1A-PPO14A nucleotide sequences, PPO-APA nucleotide sequence, PPO-ATA nucleotide sequence and the control vector were introduced**

| *Arabidopsis thaliana* genotype | Concentration (g ai/ha) | The grade of pesticide damage/plant | | | |
|---|---|---|---|---|---|
| | | 0 | 1 | 2 | 3 |
| CK | 0 | 24 | 0 | 0 | 0 |
| | 25 | 0 | 0 | 0 | 24 |
| | 100 | 0 | 0 | 0 | 24 |
| Control vector | 0 | 24 | 0 | 0 | 0 |
| | 25 | 0 | 0 | 0 | 24 |
| | 100 | 0 | 0 | 0 | 24 |
| PPO1A | 0 | 24 | 0 | 0 | 0 |
| | 25 | 24 | 0 | 0 | 0 |
| | 100 | 4 | 20 | 0 | 0 |
| PPO2A | 0 | 24 | 0 | 0 | 0 |
| | 25 | 24 | 0 | 0 | 0 |
| | 100 | 6 | 18 | 0 | 0 |
| PPO3A | 0 | 24 | 0 | 0 | 0 |
| | 25 | 24 | 0 | 0 | 0 |
| | 100 | 2 | 22 | 0 | 0 |
| PPO4A | 0 | 24 | 0 | 0 | 0 |
| | 25 | 24 | 0 | 0 | 0 |
| | 100 | 4 | 20 | 0 | 0 |
| PPO5A | 0 | 24 | 0 | 0 | 0 |
| | 25 | 24 | 0 | 0 | 0 |
| | 100 | 0 | 24 | 0 | 0 |
| PPO6A | 0 | 24 | 0 | 0 | 0 |
| | 25 | 24 | 0 | 0 | 0 |
| | 100 | 2 | 22 | 0 | 0 |
| PPO7A | 0 | 24 | 0 | 0 | 0 |
| | 25 | 24 | 0 | 0 | 0 |
| | 100 | 4 | 20 | 0 | 0 |
| PPO8A | 0 | 24 | 0 | 0 | 0 |
| | 25 | 24 | 0 | 0 | 0 |
| | 100 | 8 | 14 | 2 | 0 |
| PPO9A | 0 | 24 | 0 | 0 | 0 |
| | 25 | 24 | 0 | 0 | 0 |
| | 100 | 0 | 24 | 0 | 0 |
| PPO10A | 0 | 24 | 0 | 0 | 0 |
| | 25 | 24 | 0 | 0 | 0 |
| | 100 | 0 | 24 | 0 | 0 |
| PPO11A | 0 | 24 | 0 | 0 | 0 |
| | 25 | 24 | 0 | 0 | 0 |
| | 100 | 0 | 24 | 0 | 0 |
| PPO12A | 0 | 24 | 0 | 0 | 0 |
| | 25 | 24 | 0 | 0 | 0 |
| | 100 | 0 | 24 | 0 | 0 |
| PPO13A | 0 | 24 | 0 | 0 | 0 |
| | 25 | 24 | 0 | 0 | 0 |
| | 100 | 0 | 24 | 0 | 0 |
| PPO14A | 0 | 24 | 0 | 0 | 0 |
| | 25 | 24 | 0 | 0 | 0 |
| | 100 | 0 | 24 | 0 | 0 |
| PPO-APA | 0 | 24 | 0 | 0 | 0 |
| | 25 | 0 | 0 | 0 | 24 |
| | 100 | 0 | 0 | 0 | 24 |
| PPO-ATA | 0 | 24 | 0 | 0 | 0 |
| | 25 | 0 | 0 | 0 | 24 |
| | 100 | 0 | 0 | 0 | 24 |

For *Arabidopsis thaliana,* 25 g ai/ha saflufenacil herbicide is an effective dose distinguishing sensitive plants from plants having an average level of resistance. The results of Table 2 show that compared with the control vector and CK, (1) the genotypes PPO1A-PPO14A all exhibited high-resistant tolerance to saflufenacil at one-fold field concentration, while the genotypes PPO-APA and PPO-ATA both exhibited no tolerance; (2) the genotypes PPO1A-PPO7A and PPO9A-PPO14A all exhibited high-resistant tolerance to saflufenacil at four-fold field concentration (only 2 plants in the genotype PPO8A exhibited moderate- or poor-resistant tolerance, and the other 22 plants all exhibited high-resistant tolerance), while the genotypes PPO-APA and PPO-ATA both exhibited no tolerance.

**Table 3 Experimental results of the flumioxazin tolerance of Arabidopsis thaliana T₁ plants into which the PPO1A-PPO14A nucleotide sequences, PPO-APA nucleotide sequence, PPO-ATA nucleotide sequence and the control vector were introduced**

| *Arabidopsis thaliana* genotype | Concentration (g ai/ha) | The grade of pesticide damage/plant | | | |
|---|---|---|---|---|---|
| | | 0 | 1 | 2 | 3 |
| CK | 0 | 24 | 0 | 0 | 0 |
| | 60 | 0 | 0 | 0 | 24 |
| | 240 | 0 | 0 | 0 | 24 |
| Control vector | 0 | 24 | 0 | 0 | 0 |
| | 60 | 0 | 0 | 0 | 24 |
| | 240 | 0 | 0 | 0 | 24 |
| PPO1A | 0 | 24 | 0 | 0 | 0 |
| | 60 | 24 | 0 | 0 | 0 |
| | 240 | 23 | 1 | 0 | 0 |
| PPO2A | 0 | 24 | 0 | 0 | 0 |
| | 60 | 24 | 0 | 0 | 0 |
| | 240 | 24 | 0 | 0 | 0 |
| PPO3A | 0 | 24 | 0 | 0 | 0 |
| | 60 | 24 | 0 | 0 | 0 |
| | 240 | 24 | 0 | 0 | 0 |
| PPO4A | 0 | 24 | 0 | 0 | 0 |
| | 60 | 24 | 0 | 0 | 0 |
| | 240 | 22 | 2 | 0 | 0 |
| PPO5A | 0 | 24 | 0 | 0 | 0 |
| | 60 | 24 | 0 | 0 | 0 |
| | 240 | 21 | 3 | 0 | 0 |
| PPO6A | 0 | 24 | 0 | 0 | 0 |
| | 60 | 24 | 0 | 0 | 0 |
| | 240 | 22 | 2 | 0 | 0 |
| PPO7A | 0 | 24 | 0 | 0 | 0 |
| | 60 | 24 | 0 | 0 | 0 |
| | 240 | 24 | 0 | 0 | 0 |
| PPO8A | 0 | 24 | 0 | 0 | 0 |
| | 60 | 24 | 0 | 0 | 0 |
| | 240 | 23 | 1 | 0 | 0 |
| PPO9A | 0 | 24 | 0 | 0 | 0 |
| | 60 | 24 | 0 | 0 | 0 |
| | 240 | 24 | 0 | 0 | 0 |
| PPO10A | 0 | 24 | 0 | 0 | 0 |
| | 60 | 24 | 0 | 0 | 0 |
| | 240 | 24 | 0 | 0 | 0 |
| PPO11A | 0 | 24 | 0 | 0 | 0 |
| | 60 | 24 | 0 | 0 | 0 |
| | 240 | 20 | 4 | 0 | 0 |
| PPO12A | 0 | 24 | 0 | 0 | 0 |
| | 60 | 24 | 0 | 0 | 0 |
| | 240 | 24 | 0 | 0 | 0 |
| PPO13A | 0 | 24 | 0 | 0 | 0 |
| | 60 | 24 | 0 | 0 | 0 |
| | 240 | 19 | 5 | 0 | 0 |
| PPO14A | 0 | 24 | 0 | 0 | 0 |
| | 60 | 24 | 0 | 0 | 0 |
| | 240 | 19 | 5 | 0 | 0 |
| PPO-APA | 0 | 24 | 0 | 0 | 0 |
| | 60 | 0 | 0 | 0 | 24 |
| | 240 | 0 | 0 | 0 | 24 |
| PPO-ATA | 0 | 24 | 0 | 0 | 0 |
| | 60 | 0 | 0 | 2 | 22 |
| | 240 | 0 | 0 | 0 | 24 |

For *Arabidopsis thaliana,* 60 g ai/ha flumioxazin herbicide is an effective dose distinguishing sensitive plants from plants having an average level of resistance. The results of Table 3 show that compared with the control vector and CK, the genotypes PPO1A-PPO14A all exhibited high-resistant tolerance to flumioxazin at different concentrations, while the genotypes PPO-APA and PPO-ATA both exhibited basically no tolerance.

**Table 4 Experimental results of the sulfentrazone tolerance of Arabidopsis thaliana T₁ plants into which the PPO1A-PPO14A nucleotide sequences, PPO-APA nucleotide sequence, PPO-ATA nucleotide sequence and the control vector were introduced**

| *Arabidopsis thaliana* genotype | Concentration (g ai/ha) | The grade of pesticide | | | |
|---|---|---|---|---|---|
| | | 0 | 1 | 2 | 3 |
| CK | 0 | 24 | 0 | 0 | 0 |
| | 450 | 0 | 0 | 0 | 24 |
| | 900 | 0 | 0 | 0 | 24 |
| Control vector | 0 | 24 | 0 | 0 | 0 |
| | 450 | 0 | 0 | 0 | 24 |
| | 900 | 0 | 0 | 0 | 24 |
| PPO1A | 0 | 24 | 0 | 0 | 0 |
| | 450 | 24 | 0 | 0 | 0 |
| | 900 | 20 | 4 | 0 | 0 |
| PPO2A | 0 | 24 | 0 | 0 | 0 |
| | 450 | 24 | 0 | 0 | 0 |
| | 900 | 20 | 4 | 0 | 0 |
| PPO3A | 0 | 24 | 0 | 0 | 0 |
| | 450 | 24 | 0 | 0 | 0 |
| | 900 | 20 | 4 | 0 | 0 |
| PPO4A | 0 | 24 | 0 | 0 | 0 |
| | 450 | 24 | 0 | 0 | 0 |
| | 900 | 18 | 6 | 0 | 0 |
| PPO5A | 0 | 24 | 0 | 0 | 0 |
| | 450 | 24 | 0 | 0 | 0 |
| | 900 | 20 | 4 | 0 | 0 |
| PPO6A | 0 | 24 | 0 | 0 | 0 |
| | 450 | 24 | 0 | 0 | 0 |
| | 900 | 20 | 4 | 0 | 0 |
| PPO7A | 0 | 24 | 0 | 0 | 0 |
| | 450 | 24 | 0 | 0 | 0 |
| | 900 | 22 | 2 | 0 | 0 |
| PPO8A | 0 | 24 | 0 | 0 | 0 |
| | 450 | 24 | 0 | 0 | 0 |
| | 900 | 22 | 2 | 0 | 0 |
| PPO9A | 0 | 24 | 0 | 0 | 0 |
| | 450 | 24 | 0 | 0 | 0 |
| | 900 | 18 | 6 | 0 | 0 |
| PPO10A | 0 | 24 | 0 | 0 | 0 |
| | 450 | 24 | 0 | 0 | 0 |
| | 900 | 20 | 4 | 0 | 0 |
| PPO11A | 0 | 24 | 0 | 0 | 0 |
| | 450 | 24 | 0 | 0 | 0 |
| | 900 | 19 | 5 | 0 | 0 |
| PPO12A | 0 | 24 | 0 | 0 | 0 |
| | 450 | 24 | 0 | 0 | 0 |
| | 900 | 23 | 1 | 0 | 0 |
| PPO13A | 0 | 24 | 0 | 0 | 0 |
| | 450 | 24 | 0 | 0 | 0 |
| | 900 | 23 | 1 | 0 | 0 |
| PPO14A | 0 | 24 | 0 | 0 | 0 |
| | 450 | 24 | 0 | 0 | 0 |
| | 900 | 19 | 5 | 0 | 0 |
| PPO-APA | 0 | 24 | 0 | 0 | 0 |
| | 450 | 0 | 0 | 0 | 24 |
| | 900 | 0 | 0 | 0 | 24 |
| PPO-ATA | 0 | 24 | 0 | 0 | 0 |
| | 450 | 0 | 0 | 0 | 24 |
| | 900 | 0 | 0 | 0 | 24 |

For *Arabidopsis thaliana,* 450 g ai/ha sulfentrazone herbicide is an effective dose distinguishing sensitive plants from plants having an average level of resistance. The results of Table 4 show that compared with the control vector and CK, the genotypes PPO1A-PPO14A all exhibited high-resistant tolerance to sulfentrazone at different concentrations, while the genotypes PPO-APA and PPO-ATA both exhibited no tolerance.

### Example 2: Acquisition and verification of transgenic soybean plants

### 1. Transformation of Agrobacterium with the recombinant expression vectors

The recombinant expression vectors DBN12337, DBN12342, DBN12348, DBN12351, and DBN12353 (containing the PPO1A nucleotide sequence, PPO6A nucleotide sequence, PPO12A nucleotide sequence, PPO-ECA nucleotide sequence, and PPO-APA nucleotide sequence, respectively), and the control recombinant expression vector DBN12337N, as described in point 3 of Example 1, were transformed into the Agrobacterium LBA4404 (Invitrogen, Chicago, USA, CAT: 18313-015) respectively using a liquid nitrogen method, under the following transformation conditions: 100 µL of Agrobacterium LBA4404, and 3 µL of plasmid DNA (recombinant expression vector) were placed in liquid nitrogen for 10 minutes, and bathed in warm water at 37°C for 10 minutes; the transformed Agrobacterium LBA4404 were inoculated into an LB tube, cultured under the conditions of a temperature of 28°C and a rotation speed of 200 rpm for 2 hours, and then spread on the LB plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN12337, DBN12342, DBN12348, DBN12351, DBN12353 and the control recombinant expression vector DBN12337N were completely correct.

### 2. Acquisition of transgenic soybean plants

According to the conventional Agrobacterium infection method, the cotyledonary node tissue of a sterilely cultured soybean variety Zhonghuang13 was co-cultured with the Agrobacterium as described in point 1 of this Example, so as to introduce the T-DNA (comprising 34S enhancer sequence of figwort mosaic virus, promoter sequence of oilseed rape eukaryotic elongation factor gene 1α (Tsf1), *Arabidopsis thaliana* chloroplast transit peptide sequence, 5-enolpyruvylshikimate-3-phosphate synthase gene, terminator sequence of a pea RbcS gene, promoter sequence of *Arabidopsis thaliana* Ubiquitin10 gene, *Arabidopsis thaliana* albino or pale-green chloroplast transit peptide, PPO1A nucleotide sequence, PPO6A nucleotide sequence, PPO12Anucleotide sequence, PPO-ECA nucleotide sequence, PPO-APA nucleotide sequence, terminator sequence of a nopaline synthetase gene, 35S promoter sequence of a cauliflower mosaic virus, phosphinothricin-N-acetyl-transferase gene, and 35S terminator sequence of cauliflower mosaic virus) of the recombinant expression vectors DBN12337, DBN12342, DBN12348, DBN12351, DBN12353 and the control recombinant expression vector DBN12337N into the soybean chromosomes, thereby respectively obtaining soybean plants into which the PPO1A nucleotide sequence was introduced, soybean plants into which the PPO6A nucleotide sequence was introduced, soybean plants into which the PPO12A nucleotide sequence was introduced, soybean plants into which the PPO-ECA nucleotide sequence was introduced, soybean plants into which the PPO-APA nucleotide sequence was introduced, and soybean plants into which the control vector DBN12337N was introduced.

For the Agrobacterium-mediated soybean transformation, briefly, mature soybean seeds were germinated in a soybean germination culture medium (3.1 g/L of B5 salt, B5 vitamin, 20 g/L of sucrose, and 8 g/L of agar, pH 5.6), and then cultured under the conditions of a temperature of 25 ± 1°C; and a photoperiod (light/dark) of 16 h/8 h. After 4-6 days of germination, soybean sterile seedlings swelling at bright green cotyledonary nodes were taken, hypocotyledonary axes were cut off 3-4 millimeters below the cotyledonary nodes, the cotyledons were cut longitudinally, and apical buds, lateral buds and seminal roots were removed. A wound was created at a cotyledonary node using the knife back of a scalpel, and the wounded cotyledonary node tissues were contacted with an Agrobacterium suspension, wherein the Agrobacterium can transfer the PPO1A nucleotide sequence, PPO6A nucleotide sequence, PPO12A nucleotide sequence, PPO-ECA nucleotide sequence, or PPO-APA nucleotide sequence respectively to the wounded cotyledonary node tissues (step 1: the infection step). In this step, the cotyledonary node tissues were preferably immersed in the Agrobacterium suspension (OD₆₆₀ = 0.5-0.8, an infection culture medium (2.15 g/L of MS salt, B5 vitamin, 20 g/L of sucrose, 10 g/L of glucose, 40 mg/L of acetosyringone (AS), 4 g/L of 2-morpholine ethanesulfonic acid (MES), and 2 mg/L of zeatin (ZT), pH 5.3)) to initiate the inoculation. The cotyledon tissues were co-cultured with Agrobacterium for a period of time (3 days) (step 2: co-culturing step). Preferably, after the infection step, the cotyledon tissues were cultured in a solid medium (4.3 g/L MS salts, B5 vitamins, 20 g/L sucrose, 10 g/L glucose, 4 g/L MES, 2 mg/L ZT, 8 g/L agar; pH 5.6). After this co-culturing stage, there can be an optional "recovery" step in which a recovery culture medium (3.1 g/L of B5 salt, B5 vitamin, 1 g/L of MES, 30 g/L of sucrose, 2 mg/L of ZT, 8 g/L of agar, 150 mg/L of cephalosporin, 100 mg/L of glutamic acid, and 100 mg/L of aspartic acid, pH 5.6) with the addition of at least one antibiotic (150-250 mg/L of cephalosporin) for inhibiting the growth of Agrobacterium, and without the addition of a selective agent for a plant transformant, was used (step 3: recovery step). Preferably, the tissue blocks regenerated from the cotyledonary nodes were cultured in a solid medium comprising antibiotic but no selective agent, so as to eliminate Agrobacterium and provide a recovery period for the infected cells. Subsequently, the tissue blocks regenerated from the cotyledonary nodes were cultured in a culture medium containing a selective agent (glyphosate), and on-growing transformed calli were selected (step 4: the selection step). Preferably, the tissue blocks regenerated from the cotyledonary nodes were cultured in a screening solid culture medium (3.1 g/L of B5 salt, B5 vitamin, 1 g/L of MES, 30 g/L of sucrose, 1 mg/L of 6-benzyladenine (6-BAP), 8 g/L of agar, 150 mg/L of cephalosporin, 100 mg/L of glutamic acid, 100 mg/L of aspartic acid, and 0.25 mol/L of N-(phosphonomethyl)glycine, pH 5.6) containing a selective agent, thus resulting in selective growth of the transformed cells. Then, plants were regenerated from the transformed cells (step 5: the regeneration step). Preferably, the tissue blocks regenerated from the cotyledonary nodes grown in a culture medium containing a selective agent were cultured in solid culture media (a B5 differentiation culture medium and B5 rooting culture medium) to regenerate plants.

The screened out resistant tissues were transferred onto the B5 differentiation culture medium (3.1 g/L of B5 salt, B5 vitamin, 1 g/L of MES, 30 g/L of sucrose, 1 mg/L of ZT, 8 g/L of agar, 150 mg/L of cephalosporin, 50 mg/L of glutamic acid, 50 mg/L of aspartic acid, 1 mg/L of gibberellin, 1 mg/L of auxin, and 0.25 mol/L of N-(phosphonomethyl)glycine, pH 5.6), and cultured at 25°C for differentiation. The differentiated seedlings were transferred onto the B5 rooting culture medium (3.1 g/L of B5 salt, B5 vitamin, 1 g/L of MES, 30 g/L of sucrose, 8 g/L of agar, 150 mg/L of cephalosporin, and 1 mg/L of indole-3-butyric acid (IBA)), cultured in the rooting culture medium at 25°C until a height of about 10 cm, and then transferred to a glasshouse until fruiting. In the greenhouse, the plants were cultured at 26°C for 16 hours, and then cultured at 20°C for 8 hours per day.

### 3. Verification of the transgenic soybean plants using TaqMan

About 100 mg of leaves from the soybean plants into which the PPO1A nucleotide sequence was introduced, soybean plants into which the PPO6A nucleotide sequence was introduced, soybean plants into which the PPO12A nucleotide sequence was introduced, soybean plants into which the PPO-ECA nucleotide sequence was introduced, soybean plants into which the PPO-APA nucleotide sequence was introduced, and soybean plants into which the control vector DBN12337N was introduced were taken as samples, and the genomic DNA thereof was extracted with a DNeasy Plant Maxi Kit of Qiagen, and copy numbers of an EPSPS gene were detected by the Taqman probe fluorescence quantitative PCR method so as to determine the copy numbers of the PPO gene. At the same time, wild-type soybean plants were used as the control, and detected and analyzed according to the above-mentioned method. Triple repeats were set for the experiments, and the average value was calculated.

The specific method for detecting the copy number of the EPSPS gene was as follows:
Step 11: 100 mg of leaves of the soybean plants into which the PPO1A nucleotide sequence was introduced, soybean plants into which the PPO6A nucleotide sequence was introduced, soybean plants into which the PPO12A nucleotide sequence was introduced, soybean plants into which the PPO-ECA nucleotide sequence was introduced, soybean plants into which the PPO-APA nucleotide sequence was introduced, soybean plants into which the control vector DBN12337N was introduced, and wild-type soybean plants were taken, and ground into a homogenate using liquid nitrogen in a mortar, and triple repeats were taken for each sample;
Step 12: The genomic DNA of the above-mentioned samples was extracted using a DNeasy Plant Mini Kit of Qiagen, with the particular method as described in the product manual;
Step 13: The concentrations of the genomic DNA of the above-mentioned samples were detected using NanoDrop 2000 (Thermo Scientific);
Step 14: The concentrations of the genomic DNA of the above-mentioned samples were adjusted to a same value in the range of from 80 to 100 ng/µL;
Step 15: The copy numbers of the samples were identified using the Taqman probe fluorescence quantitative PCR method, wherein samples for which the copy numbers were known and had been identified were taken as standards, the samples of the wild-type soybean plants were taken as the control, and triple repeats were taken for each sample, and were averaged; the sequences of fluorescence quantitative PCR primers and a probe were as follows:
The following primers and probe were used to detect the EPSPS gene sequence:
Primer 1: ctggaaggcgaggacgtcatcaata, as set forth in SEQ ID NO: 69 in the SEQUENCE LISTING;
Primer 2: tggcggcattgccgaaatcgag, as set forth in SEQ ID NO: 70 in the SEQUENCE LISTING;
Probe 1: atgcaggcgatgggcgcccgcatccgta, as set forth in SEQ ID NO: 71 in the SEQUENCE LISTING;
PCR reaction system:

| | |
|---|---|
| JumpStart^{™} Taq ReadyMix^{™} (Sigma) | 10 µL |
| 50× primer/probe mixture | 1 µL |
| genomic DNA | 3 µL |
| water (ddH₂O) | 6 µL |

The 50× primer/probe mixture comprises 45 µL of each primer at a concentration of 1 mM, 50 µL of the probe at a concentration of 100 µM, and 860 µL of 1 × TE buffer, and was stored at 4°C in an amber tube.
PCR reaction conditions:

| Step | Temperature | Time |
|---|---|---|
| 21 | 95°C | 5 min |
| 22 | 95°C | 30 s |
| 23 | 60°C | 1 min |
| 24 | go back to step 22, and repeat 40 times | |

Data was analyzed using software SDS2.3 (Applied Biosystems).

By analyzing the experimental results of the copy number of the EPSPS gene, it was further demonstrated that the PPO 1A nucleotide sequence, PPO6A nucleotide sequence, PPO12A nucleotide sequence, PPO-ECA nucleotide sequence, PPO-APA nucleotide sequence and the control vector DBN12337N had all been incorporated into the chromosome of the detected soybean plants, and all of the soybean plants into which the PPO1A nucleotide sequence was introduced, soybean plants into which the PPO6A nucleotide sequence was introduced, soybean plants into which the PPO 12A nucleotide sequence was introduced, soybean plants into which the PPO-ECA nucleotide sequence was introduced, soybean plants into which the PPO-APA nucleotide sequence was introduced, and the soybean plants into which the control vector DBN12337N was introduced resulted in single-copy transgenic soybean plants.

### 4. Detection of the herbicide tolerance of the transgenic soybean plants

The soybean plants (PPO1A) into which the PPO1A nucleotide sequence was introduced, the soybean plants (PPO6A) into which the PPO6A nucleotide sequence was introduced, the soybean plants (PPO12A) into which the PPO12A nucleotide sequence was introduced, the soybean plants (PPO-ECA) into which the PPO-ECA nucleotide sequence was introduced, the soybean plants (PPO-APA) into which the PPO-APA nucleotide sequence was introduced, the soybean plants (control vector) into which the control vector was introduced, and the wild-type soybean plants (CK) (with 16 plants being included in each genotype) (18 days after sowing) were taken and sprayed with saflufenacil at three concentrations (50 g ai/ha (two-fold field concentration, 2×), 100 g ai/ha (four-fold field concentration, 4×), and 0 g ai/ha (water, 0×)), oxyfluorfen at three concentrations (360 g ai/ha (two-fold field concentration, 2×), 720 g ai/ha (four-fold field concentration, 4×), and 0 g ai/ha (water, 0×), and flumioxazin at three concentrations (120 g ai/ha (two-fold field concentration, 2×), 240 g ai/ha (four-fold field concentration, 4×), and 0 g ai/ha (water, 0×) to determine the tolerance of soybean plants to the herbicides. According to the method as described above in point 6 of Example 1, after 7 days of spraying (7 DAT), the damage level of each plant caused by the herbicide was evaluated according to the average of the damage levels (%) of plants. The experimental results are shown in Tables 5-7.

**Table 5 Experimental results of the saflufenacil tolerance of transgenic soybean plants**

| Soybean genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 50 | 0 | 0 | 0 | 16 |
| | 100 | 0 | 0 | 0 | 16 |
| Control vector | 0 | 16 | 0 | 0 | 0 |
| | 50 | 0 | 0 | 0 | 16 |
| | 100 | 0 | 0 | 0 | 16 |
| PPO1A | 0 | 16 | 0 | 0 | 0 |
| | 50 | 16 | 0 | 0 | 0 |
| | 100 | 16 | 0 | 0 | 0 |
| PPO6A | 0 | 16 | 0 | 0 | 0 |
| | 50 | 16 | 0 | 0 | 0 |
| | 100 | 16 | 0 | 0 | 0 |
| PPO 12A | 0 | 16 | 0 | 0 | 0 |
| | 50 | 16 | 0 | 0 | 0 |
| | 100 | 16 | 0 | 0 | 0 |
| PPO-ECA | 0 | 16 | 0 | 0 | 0 |
| | 50 | 9 | 7 | 0 | 0 |
| | 100 | 8 | 3 | 5 | 0 |
| PPO-APA | 0 | 16 | 0 | 0 | 0 |
| | 50 | 0 | 0 | 8 | 8 |
| | 100 | 0 | 0 | 0 | 16 |

The results of Table 5 show that (1) compared with the control vector and CK, the genotypes PPO1A, PPO6A, PPO12A and PPO-ECA could exhibit different extents of tolerance to saflufenacil, while PPO-APA exhibited basically no tolerance; (2) for the treatment with saflufenacil at two-fold field concentration, the damage levels in the genotypes PPO1A, PPO6A and PPO12A were rated at grade 0, while about 44% of the plants in the genotype PPO-ECA was rated at grade 1; and (3) the genotypes PPO1A, PPO6A and PPO12A all exhibited high-resistant tolerance to saflufenacil at four-fold field concentration, while about 31% of the plants in the genotype PPO-ECA exhibited moderate- or poor-resistant tolerance.

**Table 6 Experimental results of the oxyfluorfen tolerance of transgenic soybean plants**

| Soybean genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 360 | 0 | 0 | 0 | 16 |
| | 720 | 0 | 0 | 0 | 16 |
| Control vector | 0 | 16 | 0 | 0 | 0 |
| | 360 | 0 | 0 | 0 | 16 |
| | 720 | 0 | 0 | 0 | 16 |
| PPO1A | 0 | 16 | 0 | 0 | 0 |
| | 360 | 16 | 0 | 0 | 0 |
| | 720 | 16 | 0 | 0 | 0 |
| PPO6A | 0 | 16 | 0 | 0 | 0 |
| | 360 | 16 | 0 | 0 | 0 |
| | 720 | 16 | 0 | 0 | 0 |
| PPO 12A | 0 | 16 | 0 | 0 | 0 |
| | 360 | 16 | 0 | 0 | 0 |
| | 720 | 16 | 0 | 0 | 0 |
| PPO-ECA | 0 | 16 | 0 | 0 | 0 |
| | 360 | 16 | 0 | 0 | 0 |
| | 720 | 13 | 3 | 0 | 0 |
| PPO-APA | 0 | 16 | 0 | 0 | 0 |
| | 360 | 0 | 0 | 8 | 8 |
| | 720 | 0 | 0 | 0 | 16 |

The results of Table 6 show that compared with the control vector and CK, (1) the genotypes PPO1A, PPO6A, PPO12A and PPO-ECA all exhibited high-resistant tolerance to oxyfluorfen at two-fold field concentration, while 50% of the plants in the genotype PPO-APA exhibited no tolerance; and (2) the genotypes PPO1A, PPO6A, PPO12A and PPO-ECA all exhibited high-resistant tolerance to oxyfluorfen at four-fold field concentration, while the genotype PPO-APA exhibited no tolerance.

**Table 7 Experimental results of the flumioxazin tolerance of transgenic soybean plants**

| Soybean genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 120 | 0 | 0 | 0 | 16 |
| | 240 | 0 | 0 | 0 | 16 |
| Control vector | 0 | 16 | 0 | 0 | 0 |
| | 120 | 0 | 0 | 0 | 16 |
| | 240 | 0 | 0 | 0 | 16 |
| PPO1A | 0 | 16 | 0 | 0 | 0 |
| | 120 | 16 | 0 | 0 | 0 |
| | 240 | 16 | 0 | 0 | 0 |
| PPO6A | 0 | 16 | 0 | 0 | 0 |
| | 120 | 16 | 0 | 0 | 0 |
| | 240 | 16 | 0 | 0 | 0 |
| PPO 12A | 0 | 16 | 0 | 0 | 0 |
| | 120 | 16 | 0 | 0 | 0 |
| | 240 | 16 | 0 | 0 | 0 |
| PPO-ECA | 0 | 16 | 0 | 0 | 0 |
| | 120 | 16 | 0 | 0 | 0 |
| | 240 | 15 | 1 | 0 | 0 |
| PPO-APA | 0 | 16 | 0 | 0 | 0 |
| | 120 | 0 | 0 | 0 | 16 |
| | 240 | 0 | 0 | 0 | 16 |

The results of Table 7 show that compared with the control vector and CK, the genotypes PPO1A, PPO6A, PPO12A and PPO-ECA all exhibited high-resistant tolerance to flumioxazin at different concentrations, while the genotype PPO-APA exhibited no flumioxazin tolerance.

### Example 3 Acquisition and verification of transgenic maize plants

### 1. Construction of the recombinant expression vectors of maize containing PPO genes

The 5' and 3' ends of the PPO1B nucleotide sequence, the PPO6B nucleotide sequence and the PPO12B nucleotide sequence, and the PPO-APB nucleotide sequence as described in point 1 of Example 1 were respectively linked to the following universal adapter primer 2:
Universal adapter primer 2 for the 5' end: 5'-ccaagcggccaagctta-3', as set forth in SEQ ID NO: 72 in the SEQUENCE LISTING;
Universal adapter primer 2 for the 3' end: 5'-tgtttgaacgatcggcgcgcc-3', as set forth in SEQ ID NO: 73 in the SEQUENCE LISTING.

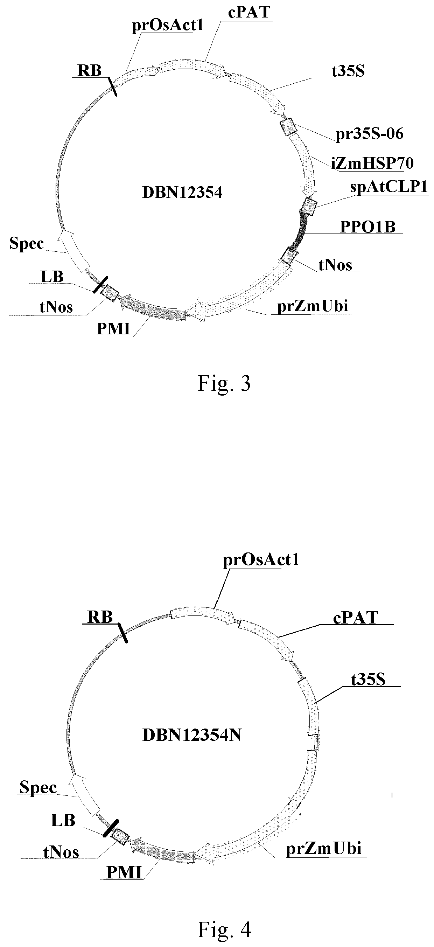

A plant expression vector DBNBC-02 was subjected to double digestion using restriction enzymes *Spe I* and *Asc I* to linearize the plant expression vector. The digestion product was purified to obtain the linearized DBNBC-02 expression vector backbone (vector backbone: pCAMBIA2301 (which is available from CAMBIA)), which then underwent a recombination reaction with the PPO1B nucleotide sequence linked to the universal adapter primer 2, according to the procedure of Takara In-Fusion products seamless connection kit (Clontech, CA, USA, CAT: 121416) instructions, to construct a recombinant expression vector BN12354 with the vector structure as shown in FIG. 3. (Spec: spectinomycin gene; RB: right border; prOsAct1: rice actin 1 promoter (SEQ ID NO:74); cPAT: phosphinothricin-N-acetyl-transferase gene (SEQ ID NO:60); t35S: cauliflower mosaic virus 35S terminator (SEQ ID NO: 61); pr35S-06: cauliflower mosaic virus 35S promoter (SEQ ID NO:75); iZmHSP70: Zea mays heat shock 70 kDa protein intron (SEQ ID NO:76); spAtCLP1: *Arabidopsis thaliana* albino or pale-green chloroplast transit peptide (SEQ ID NO:57); PPO1B: PPO1B nucleotide sequence (SEQ ID NO:62); tNos: nopaline synthetase gene terminator (SEQ ID NO:58); prZmUbi: Zea mays ubiquitin 1 gene promoter (SEQ ID NO:77); PMI: phosphomannose isomerase gene (SEQ ID NO:78); tNos: terminator of a nopaline synthase gene (SEQ ID NO: 58); LB: left border).

*Escherichia coli* T1 competent cells were transformed according to the heat shock method described in point 3 of Example 1, and the plasmids in the cells were extracted through the alkaline method. The extracted plasmid was identified by sequencing. The results indicated that the recombinant expression vector DBN12354 contained the nucleotide sequence set forth in SEQ ID NO:62 in the SEQUENCE LISTING, i.e. the PPO1B nucleotide sequence.

According to the above method for constructing recombinant expression vector DBN12354, the PPO6B nucleotide sequence, the PPO12B nucleotide sequence and the PPO-APB nucleotide sequence which were linked to the universal adapter primer 2 were respectively subjected to a recombination reaction with the linearized DBNBC-02 expression vector backbone, to construct the recombinant expression vectors DBN12355 to DBN12357 in sequence. Sequencing verified that the PPO6B nucleotide sequence, the PPO12B nucleotide sequence and the PPO-APB nucleotide sequence were correctly inserted in the recombinant expression vectors DBN12355 to DBN12357.

According to the method for constructing the recombinant expression vector DBN12354 as described above, the recombinant expression vector DBN12354N was constructed as the control, and its structure was shown in FIG. 4 (Spec: the spectinomycin gene; RB: right border; prOsAct1: rice actin 1 promoter (SEQ ID NO:74); cPAT: phosphinothricin-N-acetyl-transferase gene (SEQ ID NO:60); t35S: cauliflower mosaic virus 35S terminator (SEQ ID NO: 61); prZmUbi: Zea mays ubiquitin 1 gene promoter (SEQ ID NO:77); PMI: phosphomannose isomerase gene (SEQ ID NO:78); tNos: terminator of a nopaline synthase gene (SEQ ID NO: 58); LB: left border).

### 2. Transformation of Agrobacterium with the recombinant expression vectors

The recombinant expression vectors DBN12354 to DBN12357 which had been correctly constructed, and the above-mentioned control recombinant expression vector DBN12354N, were transformed respectively into the Agrobacterium LBA4404 (Invitrogen, Chicago, USA, CAT: 18313-015) using a liquid nitrogen method, under the following transformation conditions: 100 µL of Agrobacterium LBA4404, and 3 µL of plasmid DNA (recombinant expression vector) were placed in liquid nitrogen for 10 minutes, and bathed in warm water at 37°C for 10 minutes; the transformed Agrobacterium LBA4404 were inoculated into an LB tube, cultured under the conditions of a temperature of 28°C and a rotation speed of 200 rpm for 2 hours, and then spread on the LB plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN12354 to DBN12357 and the control vector DBN12354N were completely correct.

### 3. Acquisition of transgenic maize plants

According to the Agrobacterium infection method conventionally used, young embryos of a sterilely cultured maize variety Zong31 (Z31) were co-cultured with the Agrobacterium as described in point 2 of this Example, so as to introduce T-DNA (comprising rice actin 1 promoter sequence; phosphinothricin-N-acetyl-transferase gene; cauliflower mosaic virus 35S terminator sequence; cauliflower mosaic virus 35S promoter sequence; Zea mays heat shock 70 kDa protein intron sequence; *Arabidopsis thaliana* albino or pale-green chloroplast transit peptide; PPO1B nucleotide sequence; PPO6B nucleotide sequence; PPO12B nucleotide sequence; PPO-APB nucleotide sequence; nopaline synthetase gene terminator sequence; Zea mays ubiquitin 1 gene promoter sequence; phosphomannose isomerase gene; and terminator sequence of a nopaline synthase gene) of the recombinant expression vectors DBN12354 to DBN12357 and the control recombinant expression vector DBN12354N as constructed in point 1 of this Example into the maize chromosomes, respectively obtaining maize plants into which the PPO1B nucleotide sequence was introduced, maize plants into which the PPO6B nucleotide sequence was introduced, maize plants into which the PPO12B nucleotide sequence was introduced, maize plants into which the PPO-APB nucleotide sequence was introduced, and maize plants into which the control vector DBN12354N was introduced; meanwhile, wild-type maize plants were used as the control.

For the Agrobacterium-mediated maize transformation, briefly, immature young embryos were separated from maize, and contacted with an Agrobacterium suspension, wherein the Agrobacterium can transfer the PPO1B nucleotide sequence, the PPO6B nucleotide sequence and the PPO12B nucleotide sequence or PPO-APB nucleotide sequence to at least one cell of one of young embryos (step 1: infection step). In this step, the young embryos were preferably immersed in the Agrobacterium suspension (OD₆₆₀ =0.4- 0.6, infection medium (4.3 g/L MS salts, N6 vitamins, 300 mg/L casein, 68.5 g/L sucrose, 36 g/L glucose, 40 mg/L AS, 1 mg/L 2,4-D; pH 5.3)) to initiate the inoculation. The young embryos were co-cultured with Agrobacterium for a period of time (3 days) (step 2: co-culturing step). Preferably, after the infection step, the young embryos were cultured in a solid medium (4.3 g/LMS salts, MS vitamins, 300 mg/L casein, 20 g/L sucrose, 10 g/L glucose, 100 mg/L AS, 1 mg/L 2,4-D, 8 g/L agar; pH 5.8). After the co-culturing step, there may be a "recovery" step in which a recovery medium (4.3 g/L MS salts, MS vitamins, 300 mg/L casein, 30 g/L sucrose, 1 mg/L 2,4-D, 3 g/L plant gel; pH 5.8) with the addition of at least one antibiotic (cephamycin) for inhibiting the growth of Agrobacterium, and without the addition of any selective agent for plant transformants, was used (step 3: recovery step). Preferably, the young embryos were cultured in a solid medium comprising antibiotic but no selective agent, so as to eliminate Agrobacterium and provide a recovery period for the infected cells. Then, the inoculated young embryos were cultured on a medium containing a selective agent (mannose) and the on-growing transformed calli were selected (step 4: selection step). Preferably, the young embryos were cultured in a solid selective medium (4.3 g/L MS salts, MS vitamins, 300 mg/L casein, 30 g/L sucrose, 12.5 g/L of mannose, 1 mg/L 2,4-D, 3 g/L plant gel; pH 5.8) comprising a selective agent, which resulted in the selective growth of the transformed cells. Then, the calli was regenerated into plants (step 5: regeneration step). Preferably, the calli growing on the medium containing a selective agent were cultured in a solid medium (MS differential medium and MS rooting medium) to regenerate plants.

The resistant calli obtained from screening were transferred to MS differential medium (4.3 g/L MS salts, MS vitamins, 300 mg/L casein, 30 g/L sucrose, 2 mg/L of 6-benzyladenine, 5 mg/L of mannose, 3 g/L plant gel; pH 5.8), and cultured for differentiation at 25°C. The differentiated plantlets were transferred to MS rooting medium (2.15 g/L MS salts, MS vitamins, 300 mg/L casein, 30 g/L sucrose, 1mg/L indole-3-acetic acid, 3 g/L plant gel; pH 5.8), and cultured at 25°C. When the plantlets reached about 10 cm in height, they were moved to greenhouse and cultured until fruiting. In the greenhouse, the plants were cultured at 28°C for 16 hours, and then cultured at 20°C for 8 hours per day.

### 4. Verification of the Transgenic Maize Plants Using TaqMan

According to the method as described in point 3 of Example 2 for verifying the transgenic soybean plants using TaqMan, the maize plants into which the PPO1B nucleotide sequence was introduced, the maize plants into which the PPO6B nucleotide sequence was introduced, the maize plants into which the PPO12B nucleotide sequence was introduced, the maize plants into which the PPO-APB nucleotide sequence was introduced, and the maize plants into which the control vector DBN12354N was introduced, were detected and analyzed. The copy number of the PMI gene was detected by the Taqman probe fluorescence quantitative PCR method so as to determine the copy number of the PPO gene. In the meantime, wild-type maize plants were used as the control, and detected and analyzed according to the above-mentioned method. Triple repeats were set for the experiments, and the average value was calculated.

The following primers and probe were used to detect the PMI gene sequence:
Primer 3: gctgtaagagcttactgaaaaaattaaca, as set forth in SEQ ID NO: 79 in the SEQUENCE LISTING;
Primer 4: cgatctgcaggtcgacgg, as set forth in SEQ ID NO: 80 in SEQUENCE LISTING;
Probe 2: tctcttgctaagctgggagctcgatcc, as set forth in SEQ ID NO: 81 in the SEQUENCE LISTING.

It was further confirmed by analyzing the experimental results of the copy number of the PMI gene that the PPO1B nucleotide sequence, the PPO6B nucleotide sequence, the PPO12B nucleotide sequence, the PPO-APB nucleotide sequence and the control vector DBN12354N had all been incorporated into the chromosomes of the detected maize plants; and the maize plants into which the PPO1B nucleotide sequence was introduced, the maize plants into which the PPO6B nucleotide sequence was introduced, the maize plants into which the PPO12B nucleotide sequence was introduced, the maize plants into which the PPO-APB nucleotide sequence was introduced, and the maize plants into which the control vector DBN12354N was introduced, all resulted in single copy transgenic maize plants.

### 5. Detection of the herbicide tolerance of the transgenic maize plants

The maize plants (PPO1B) into which the PPO1B nucleotide sequence was introduced, the maize plants (PPO6B) into which the PPO6B nucleotide sequence was introduced, the maize plants (PPO 12B) into which the PPO 12B nucleotide sequence was introduced, the maize plants (PPO-APB) into which the PPO-APB nucleotide sequence was introduced, the maize plants (control vector) into which the control vector was introduced, and the wild-type maize plants (CK) (with 16 plants being included in each genotype) (18 days after sowing) were taken and sprayed with saflufenacil at three concentrations (50 g ai/ha (two-fold field concentration, 2×), 100 g ai/ha (four-fold field concentration, 4×), and 0 g ai/ha (water, 0×)), oxyfluorfen at three concentrations (360 g ai/ha (two-fold field concentration, 2×), 720 g ai/ha (four-fold field concentration, 4×), and 0 g ai/ha (water, 0×), and flumioxazin at three concentrations (120 g ai/ha (two-fold field concentration, 2×), 240 g ai/ha (four-fold field concentration, 4×), and 0 g ai/ha (water, 0×) to determine the tolerance of maize plants to the herbicides. According to the method as described above in point 6 of Example 1, after 7 days of spraying (7 DAT), the damage level of each plant caused by the herbicide was evaluated according to the average of the damage levels (%) of plants (the average of the damage levels (%) of plants = the injured leaf area / the total leaf area × 100%). The experimental results are shown in Tables 8-10.

**Table 8 Experimental results of the saflufenacil tolerance of transgenic maize plants**

| Maize genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 50 | 0 | 0 | 0 | 16 |
| | 100 | 0 | 0 | 0 | 16 |
| Control vector | 0 | 16 | 0 | 0 | 0 |
| | 50 | 0 | 0 | 0 | 16 |
| | 100 | 0 | 0 | 0 | 16 |
| PPO1B | 0 | 16 | 0 | 0 | 0 |
| | 50 | 16 | 0 | 0 | 0 |
| | 100 | 16 | 0 | 0 | 0 |
| PPO6B | 0 | 16 | 0 | 0 | 0 |
| | 50 | 16 | 0 | 0 | 0 |
| | 100 | 16 | 0 | 0 | 0 |
| PPO 12B | 0 | 16 | 0 | 0 | 0 |
| | 50 | 16 | 0 | 0 | 0 |
| | 100 | 16 | 0 | 0 | 0 |
| PPO-APB | 0 | 16 | 0 | 0 | 0 |
| | 50 | 0 | 0 | 7 | 9 |
| | 100 | 0 | 0 | 1 | 15 |

The results of Table 8 show that compared with the control vector and CK, (1) the genotypes PPO1B, PPO6B and PPO12B all exhibited high-resistant tolerance to saflufenacil at two-fold field concentration, while about 56% of the plants in the genotype PPO-APB exhibited no tolerance; (2) the genotypes PPO1B, PPO6B and PPO12B all exhibited high-resistant tolerance to saflufenacil at four-fold field concentration, while the genotype PPO-APB exhibited basically no tolerance.

**Table 9 Experimental results of the oxyfluorfen tolerance of transgenic maize plants**

| Maize genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 360 | 0 | 0 | 0 | 16 |
| | 720 | 0 | 0 | 0 | 16 |
| Control vector | 0 | 16 | 0 | 0 | 0 |
| | 360 | 0 | 0 | 0 | 16 |
| | 720 | 0 | 0 | 0 | 16 |
| PPO1B | 0 | 16 | 0 | 0 | 0 |
| | 360 | 16 | 0 | 0 | 0 |
| | 720 | 16 | 0 | 0 | 0 |
| PPO6B | 0 | 16 | 0 | 0 | 0 |
| | 360 | 16 | 0 | 0 | 0 |
| | 720 | 16 | 0 | 0 | 0 |
| PPO12B | 0 | 16 | 0 | 0 | 0 |
| | 360 | 16 | 0 | 0 | 0 |
| | 720 | 16 | 0 | 0 | 0 |
| PPO-APB | 0 | 16 | 0 | 0 | 0 |
| | 360 | 0 | 0 | 8 | 8 |
| | 720 | 0 | 0 | 0 | 16 |

The results of Table 9 show that compared with the control vector and CK, (1) the genotypes PPO1B, PPO6B and PPO12B all exhibited high-resistant tolerance to oxyfluorfen at two-fold field concentration, while about 50% of the plants in the genotype PPO-APB exhibited no tolerance; (2) the genotypes PPO1B, PPO6B and PPO12B all exhibited high-resistant tolerance to oxyfluorfen at four-fold field concentration, while the genotype PPO-APB exhibited no tolerance.

**Table 10 Experimental results of the flumioxazin tolerance of transgenic maize plants**

| Maize genotype | Concentration (g ai/ha) | The grade of pesticide damage/plant | | | |
|---|---|---|---|---|---|
| | | 0 | 1 | 2 | 3 |
| CK | 0 | 16 | 0 | 0 | 0 |
| | 120 | 0 | 0 | 0 | 16 |
| | 240 | 0 | 0 | 0 | 16 |
| Control vector | 0 | 16 | 0 | 0 | 0 |
| | 120 | 0 | 0 | 0 | 16 |
| | 240 | 0 | 0 | 0 | 16 |
| PPO1B | 0 | 16 | 0 | 0 | 0 |
| | 120 | 16 | 0 | 0 | 0 |
| | 240 | 16 | 0 | 0 | 0 |
| PPO6B | 0 | 16 | 0 | 0 | 0 |
| | 120 | 16 | 0 | 0 | 0 |
| | 240 | 16 | 0 | 0 | 0 |
| PPO12B | 0 | 16 | 0 | 0 | 0 |
| | 120 | 16 | 0 | 0 | 0 |
| | 240 | 16 | 0 | 0 | 0 |
| PPO-APB | 0 | 16 | 0 | 0 | 0 |
| | 120 | 0 | 0 | 3 | 13 |
| | 240 | 0 | 0 | 0 | 16 |

The results of Table 10 show that compared with the control vector and CK, the genotypes PPO1B, PPO6B and PPO12B all exhibited high-resistant tolerance to flumioxazin at different concentrations, while the genotype PPO-APB exhibited basically no tolerance to flumioxazin.

In summary, with regard to the plants, the protoporphyrinogen oxidases PPO1-PPO14 of the present invention can confer good tolerance to PPO-inhibitor herbicides upon the *Arabidopsis thaliana* plants; in particular, PPO1, PPO6 and PPO12 can confer good tolerance to PPO-inhibitor herbicides upon the *Arabidopsis thaliana,* soybean, and maize plants. Thus, the protoporphyrinogen oxidases PPO1-PPO14 can confer good tolerance upon the plants. With regard to the herbicides, the present invention discloses for the first time that the protoporphyrinogen oxidases PPO1-PPO14 can confer higher tolerance to PPO-inhibitor herbicides upon plants, to such an extent that the plants can tolerate at least four-fold field concentration of oxyfluorfen, saflufenacil or flumioxazin, and two-fold field concentration of sulfentrazone. Therefore, the present invention has a broad application prospect in plants.

At last, it should be noted that all the above Examples are only used to illustrate the embodiments of the present invention rather than to limit the present invention. Although the present invention is described in detail with reference to the preferred Examples, those skilled in the art should understand that the embodiments of the present invention could be modified or substituted equivalently without departing from the spirit and scope of the technical solutions of the present invention.

## Claims

1. A method for controlling weeds, **characterized by** comprising applying a herbicide containing an effective dose of a PPO inhibitor to a field where at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1-14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats, wheat, barley, millet, corn, sorghum, *Brachypodium distachyon,* rice, tobacco, sunflower, alfalfa, soybean, *Cicer arietinum*, peanut, sugar beet, cucumber, cotton, oilseed rape, potato, tomato or *Arabidopsis thaliana;* further preferably, the transgenic plant is a glyphosate-tolerant plant, and the weeds are glyphosate-resistant weeds;
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

2. The method for controlling weeds according to claim 1, **characterized in that** the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.

3. The method for controlling weeds according to claim 1 or 2, **characterized in that** the transgenic plant further comprises at least one second polynucleotide encoding a second herbicide-tolerant protein, which is different from the polynucleotide sequence encoding the protoporphyrinogen oxidase.

4. The method for controlling weeds according to claim 3, **characterized in that** the second polynucleotide encodes a selectable marker protein, a protein with synthetic activity, a protein with degradation activity, a biotic stress-resistant protein, an abiotic stress-resistant protein, a male sterility protein, a protein that affects plant yield and/or a protein that affects plant quality.

5. The method for controlling weeds according to claim 4, **characterized in that** the second polynucleotide encodes 5-enolpyruvylshikimate-3-phosphate synthase, glyphosate oxidoreductase, glyphosate-N-acetyltransferase, glyphosate decarboxylase, glufosinate acetyltransferase, alpha-ketoglutarate-dependent dioxygenase, dicamba monooxygenase, 4-hydroxy phenyl pyruvate dioxygenase, acetolactate synthase, and/or cytochrome-like protein.

6. The method for controlling weeds according to any one of claims 1-5, **characterized in that** the herbicide containing an effective dose of a PPO inhibitor further includes a glyphosate herbicide, glufosinate herbicide, auxin-like herbicide, graminicide, pre-emergence selective herbicide and/or post-emergence selective herbicide.

7. A planting combination for controlling the growth of weeds, comprising a PPO-inhibitor herbicide and at least one transgenic plant, wherein the herbicide containing an effective dose of a PPO inhibitor is applied to a field where the at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase; wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1- 14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats, wheat, barley, millet, corn, sorghum, *Brachypodium distachyon,* rice, tobacco, sunflower, alfalfa, soybean, *Cicer arietinum*, peanut, sugar beet, cucumber, cotton, oilseed rape, potato, tomato or *Arabidopsis thaliana;* further preferably, the transgenic plant is a glyphosate-tolerant plant, and the weeds are glyphosate-resistant weeds.
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

8. The planting combination for controlling the growth of weeds according to claim 7, **characterized in that** the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.

9. The planting combination for controlling the growth of weeds according to claim 7 or 8, **characterized in that** the transgenic plant further comprises at least one second polynucleotide encoding a second herbicide-tolerant protein, which is different from the polynucleotide sequence encoding the protoporphyrinogen oxidase.

10. The planting combination for controlling the growth of weeds according to claim 9, **characterized in that** the second polynucleotide encodes a selectable marker protein, a protein with synthetic activity, a protein with degradation activity, a biotic stress-resistant protein, an abiotic stress-resistant protein, a male sterility protein, a protein that affects plant yield and/or a protein that affects plant quality.

11. The planting combination for controlling the growth of weeds according to claim 10, **characterized in that** the second polynucleotide encodes 5-enolpyruvylshikimate-3-phosphate synthase, glyphosate oxidoreductase, glyphosate-N-acetyltransferase, glyphosate decarboxylase, glufosinate acetyltransferase, alpha-ketoglutarate-dependent dioxygenase, dicamba monooxygenase, 4-hydroxy phenyl pyruvate dioxygenase, acetolactate synthase, and/or cytochrome-like protein.

12. The planting combination for controlling the growth of weeds according to any one of claims 7-11, **characterized in that** the herbicide containing an effective dose of a PPO inhibitor further includes a glyphosate herbicide, glufosinate herbicide, auxin-like herbicide, graminicide, pre-emergence selective herbicide and/or post-emergence selective herbicide.

13. A method for generating a plant which is tolerant to a PPO-inhibitor herbicide, **characterized in that** the method comprises introducing a polynucleotide sequence encoding a protoporphyrinogen oxidase into the genome of the plant, and when the herbicide containing an effective dose of a PPO inhibitor is applied to a field where at least the plant is present, the plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs:1-14;
preferably, the protoporphyrinogen oxidase has at least 95% sequence identity to an amino acid sequence selected from the group consisting of SEQ ID NOs:1-14;
more preferably, the protoporphyrinogen oxidase has at least 99% sequence identity to an amino acid sequence selected from the group consisting of SEQ ID NOs:1-14;
further preferably, the protoporphyrinogen oxidase is selected from the amino acid sequences of the group consisting of SEQ ID NOs:1-14;
preferably, the introduction method comprises genetic transformation, genome editing or gene mutation methods;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

14. A method for cultivating a plant which is tolerant to a PPO-inhibitor herbicide, **characterized by** comprising:
planting at least one plant propagule, wherein the plant propagule comprises in its genome a polynucleotide encoding a protoporphyrinogen oxidase, wherein the protoporphyrinogen oxidase has at least 88% sequence identity to an amino acid sequence selected from the group consisting of SEQ ID NOs: 1-14;
allowing the plant propagule to grow into a plant; and
applying the herbicide comprising an effective dose of a PPO inhibitor to a field comprising at least the plant, and harvesting the plant having a reduced plant damage and/or increased plant yield compared with other plants without the polynucleotide sequence encoding the protoporphyrinogen oxidase;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1- 14;
further preferably, the protoporphyrinogen oxidase is selected from the amino acid sequences of the group consisting of SEQ ID NOs: 1-14;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

15. A method for protecting a plant from damages caused by a PPO-inhibitor herbicide or for conferring tolerance to the PPO-inhibitor herbicide upon a plant, **characterized by** comprising applying the herbicide comprising an effective dose of a PPO inhibitor to a field where at least one transgenic plant is present, wherein the transgenic plant comprises in its genome a polynucleotide sequence encoding a protoporphyrinogen oxidase, and the transgenic plant has a reduced plant damage and/or increased plant yield compared with other plants without the polynucleotide sequence encoding the protoporphyrinogen oxidase, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1- 14;
further preferably, the protoporphyrinogen oxidase is selected from the amino acid sequences of the group consisting of SEQ ID NOs: 1-14;
preferably, the transgenic plant comprises monocotyledonous plants and dicotyledonous plants; more preferably, the transgenic plant is oats, wheat, barley, millet, corn, sorghum, *Brachypodium distachyon,* rice, tobacco, sunflower, alfalfa, soybean, *Cicer arietinum*, peanut, sugar beet, cucumber, cotton, oilseed rape, potato, tomato or *Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

16. Use of a protoporphyrinogen oxidase for conferring tolerance to a PPO-inhibitor herbicide upon a plant, wherein the protoporphyrinogen oxidase has at least 88% sequence identity to an amino acid sequence selected from the group consisting of SEQ ID NOs: 1-14;
preferably, the protoporphyrinogen oxidase has at least 90% sequence identity to an amino acid sequence selected from the group consisting of SEQ ID NOs: 1-14;
preferably, the protoporphyrinogen oxidase has at least 95% sequence identity to an amino acid sequence selected from the group consisting of SEQ ID NOs: 1-14;
more preferably, the protoporphyrinogen oxidase has at least 99% sequence identity to an amino acid sequence selected from the group consisting of SEQ ID NOs: 1- 14;
further preferably, the protoporphyrinogen oxidase is selected from the amino acid sequences of the group consisting of SEQ ID NOs: 1-14;
preferably, the use of the protoporphyrinogen oxidase for conferring tolerance to a PPO-inhibitor herbicide upon a plant comprises applying the herbicide containing an effective dose of a PPO inhibitor to a field where at least one transgenic plant is present, wherein the transgenic plant comprises in its genome a polynucleotide sequence encoding the protoporphyrinogen oxidase, and the transgenic plant has a reduced plant damage and/or increased plant yield compared with other plants without the polynucleotide sequence encoding the protoporphyrinogen oxidase;
preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
preferably, the PPO-inhibitor herbicide comprises a PPO-inhibitor herbicide from the class of diphenyl ethers, oxadiazolones, N-phenylphthalimides, oxazolinones, phenylpyrazoles, uracils, thiadiazoles, triazolinones and/or triazinones;
further preferably, the PPO-inhibitor herbicide comprises oxyfluorfen, saflufenacil, sulfentrazone, and/or flumioxazin.

17. The use of a protoporphyrinogen oxidase for conferring tolerance to a PPO-inhibitor herbicide upon a plant according to claim 16, **characterized in that** the polynucleotide sequence of the protoporphyrinogen oxidase comprises:
(a) a polynucleotide sequence encoding an amino acid sequence having at least 88% sequence identity to a sequence selected from SEQ ID NOs: 1-14, wherein the polynucleotide sequence does not include SEQ ID NOs: 15-28; or
(b) a polynucleotide sequence as shown in any one of SEQ ID NOs: 29-42 or SEQ ID NOs: 62-64.
